# EUROPEAN PATENT SPECIFICATION

(11) **EP 1 052 265 B1**
(45) Date of publication and mention of the grant of the patent: **07.12.2005**
(21) Application number: 99900359.3
(22) Date of filing: 19.01.1999
(51) Int. Cl.: C07H 19/16, C07H 19/19

(54) **PROCESS FOR PRODUCING NUCLEOSIDE DERIVATIVES**
VERFAHREN ZUR HERSTELLUNG VON NUKLEOSID-DERIVATEN
PROCEDE SERVANT A PREPARER DES DERIVES DE NUCLEOSIDES

(30) Priority: 30.01.1998 JP 1913898
(43) Date of publication of application: 15.11.2000
(73) Proprietor: Ajinomoto Co., Inc., Tokyo 104-8315 (JP)
(72) Inventor: TAKAMATSU, Satoshi, Ajinomoto Co., Inc., Kawasaki-shi, Kanagawa-ken 210-0801 (JP); KATAYAMA, Satoshi, Ajinomoto Co., Inc., Kawasaki-shi, Kanagawa-ken 210-0801 (JP); HIROSE, Naoko, Ajinomoto Co., Inc., Kawasaki-shi, Kanagawa-ken 210-0801 (JP); IZAWA, Kunisuke, Ajinomoto Co., Inc., Kawasaki-shi, Kanagawa-ken 210-0801 (JP); MARUYAMA, Tokumi, Tokushimi-shi, Tokushima-ken 771-4262 (JP)
(74) Representative: Nicholls, Kathryn Margaret
(86) International application number: PCT/JP1999/000169
(87) International publication number: WO 1999/038879

(56) References cited:
- EP-A- 0 219 829
- JP-A- 3 170 496
- JP-A- 5 505 815
- US-A- 5 525 720
- SIDDIQUI M.A. et al., "A New Synthetic Approach to the Clinically Useful, Anti-HIV-Active Nucleoside, 9-(2,3-Dideoxy-2-Fluoro-beta-D-Threo-Pentof uranosyl)Adenine (beta-FddA). Introduction of a 2'-beta-Fluoro Substituent via Inversion of a Readily Obtainable 2'-alpha-Fluoro Isomer", TETRAHEDRON LETTERS, Vol. 39, No. 13, (26 March 1998), p. 1657-1660, XP004108441
- LESIAK K. et al., "Synthesis of Nonhydrolyzable Analogues of Thiazole-4-Carboxamide and Benzamide Adenine Dinucleotide Containing Fluorine Atom at the C2' of Adenine Nucleoside: Induction of K562 Differentiation and Inosine Monophosphate Dehydrogenase Inhibitory Activity", J. MED. CHEM., Vol. 40, (1997), p. 2533-2538, XP002920574
- MARQUEZ V.E. et al.; "Acid-Stable 2'-Fluoro Purine Dideoxynucleosides as Active Agents Against HIV", J. MED. CHEM., Vol. 33, (1990), p. 978-985, XP002109683
- JOSEPH J. BARCHI Jr. et al., "Potential Anti-AIDS Drugs. Lipophilic, Adenosine Deaminase-Activated Prodrugs", J. MED. CHEM., Vol. 34, (1991), p. 1647-1655, XP002920575
- CHU C.K. et al., "Synthesis and Structure-Activity Relationships of 6-Substituted 2',3'-Dideoxypurine Nucleosides as Potential Anti-Human Immunodeficiency Virus Agents", J. MED. CHEM., Vol. 33, (1990), p. 1553-1561, XP002920576
- KOBYLINSKAYA V.I. et al., "Synthesis of Fluoro and Azido Derivatives of Purine Nucleosides from Nucleoside 2',3'-Cyclic Sulfates", BIOORG. KHIM., Vol. 20, No. 11, (1994), p. 1226-1230, XP002920577
- ROBINS M.J. et al., "Nucleic Acid Related Compounds. 91. Biomimetic Reactions are in Harmony with Loss of 2'-Substituents as Free Radicals (Not Anions) During Mechanism-Based Inactivations of Ribonucleotide Reductases. Differential Interactions of Azide, Halogen and Alkylthio Groups with Tributylstannane and Triphenylsilane", JOURNAL OF THE AMERICAN CHEMICAL SOCIETY, Vol. 118, No. 46, (20 November 1996), p. 11341-11348, XP002920578

## Description

### TECHNICAL FIELD

The present invention relates to a novel process for producing nucleoside derivatives, more precisely to a novel process for producing nucleoside derivatives wherein a fluorine atom, etc. has been substituted for the 2'-position thereof and the 3'-position thereof has been deoxylated (de-hydroxylated; deoxidized), such as 9- (2,3-dideoxy-2-fluoro-β-D-threo-pentofuranosyl) adenine (FddA) useful as an anti-viral agent, etc., which are useful in the fields of the pharmaceutical products and the other products, to a process for producing intermediates important for the productions thereof and to the novel intermediates.

### BACKGROUND ART

Nucleoside derivatives are used or expected in the pharmaceutical and the other fields for their various applications.

Particularly in the pharmaceutical field, it is reported that 9-(2,3-dideoxy-2-fluoro-β-D-threopentofuranosyl) adenine (FddA) has a strong anti-viral activity against human immunodeficiency virus (HIV) and is greatly effective for treatment of acquired immune deficiency syndrome (AIDS) (see P. Herdewijn et al., J. Med. Chem., (30), page 2131, 1987), and many clinical tests using it for the treatment of AIDS and AIDS-related complications (ARC) are being made at present. Recently, in addition, reported are FddA derivatives as modified at the nucleic acid base site to improve their potency (see C.K. Chu et al., J. Med. Chem., (37), page 821, 1994; J.S. Driscoll et al., J. Med. Chem., (39), page 1619, 1996; C.K. Chu et al., J. Med. Chem., (39), page 4676, 1996). In view of these circumstances, the compound wherein the 2'-position of the saccharide (sugar) moiety in the purine nucleoside is replaced by a fluorine atom, etc. and the 3'-position thereof is deoxylated, is important for an anti-viral treating agent.

For processes for synthesizing FddA which have been known, there are two types of processes in a broad way. For the one type, there are processes for combining a sugar derivative wherein a fluorine atom has been introduced, with a nucleic acid base (see V.E. Marquez et al., Synthesis, (11), page 1005, 1991; V.E. Marquez et al., J. Med. Chem., (33), page 978, 1990; K. Shanmuganathan et al., J. med. Chem., (37), page 821, 1994). EP-A-0 428 109 also discloses such a process, followed by protection/deprotection and 3'-deoxylation steps. For the another one there are processes for fluorinating the 2'-position thereof using a nucleic acid derivative as a starting material (see P. Herdewijn et al., J. Med. Chem., (30), page 2131, 1987; K.W.Pankiewicz et al., J. Org. Chem., (57), page 553, 1992; H. Shiragami et al., Nucleosides & Nucleotides, (11), page 391, 1992; T. Maruyama et al., Chem. Pharm. Bull., (44), page 2331, 1996).

In the processes for the former type, many stages of troublesome operations or steps are requested to synthesize a saccharide (sugar) derivative wherein a fluorine atom has been introduced thereinto. In addition, α-anomer and β-anomer are produced when a saccharide (sugar) derivative and a nucleic acid base are combined together, and accordingly, the yield thereof is low and thereafter a step for separation thereof is needed. In view of the above, the processes are not necessarily advantageous industrially.

On the other hand, in the processes of the latter type without the process of Maruyama et al., the yield for fluorination is low and these processes are also not advantageous industrially. And, in Maruyama et al. a fluorine atom can be introduced in a good yield by using a substrate wherein a chlorine atom has been introduced thereto at the moiety of the nucleic acid base, and in this case the yield of the starting material for fluorination is low. In addition, since functional groups in the substrate are not protected individually, finally, for example, in synthesizing FddA, etc., a protection for the side chain thereof is again needed in order to deoxylate selectively the hydroxyl group at the 3'-position thereof. Accordingly, it is extremely troublesome to produce an intended compound industrially by means of the process.

### PROBLEM TO BE SOLVED OBJECT ETC.

Given the situation as above, it is desired to develop an inexpensive process for producing nucleoside derivatives including 9-(2,3-dideoxy-2-fluoro-β-D-threo-pentofuranosyl) adenine (FddA) and its related compounds, which are used or expected for their various applications in the pharmaceutical products such as an anti-viral agent and the other products, in a simplified manner and at a high yield, in particular, an economical and industrial process for producing those nucleoside derivatives capable of introducing efficiently a desired substituent group to a nucleoside derivative at the 2'-position, and also deoxylating easily the hydroxyl group at the 3'-position thereof.

Accordingly, the object of the present invention is to develop and provide a profitableprocess for producing simply and industrially the nucleoside derivatives and their related compounds including their intermediates therefor noted above, and also to develop and provide novel intermediates usable therefor.

### DISCLOSURE OF INVENTION

The present inventors have assiduously studied in order to solve the problem noted above, and, as a result, have found that, in a production of the nucleoside derivative wherein a fluorine atom, etc has been substituted for the 2'-position thereof and the hydorxyl group at the 3'-position thereof has been deoxylated (deoxidized), such as FddA, etc., the 2'-position of the saccharide (sugar) moiety can be replaced in a high yield, by substituting a halogen atom for the 6-position in the nucleic acid (a purine riboside) and modifying the hydroxyl groups at the 3'-position and 5'-position of the sugar moiety thereof with the protecting (protective) groups which can not be deprotected (deblocked) under the same condition, and completed the present invention based on this finding.

Particularly, by subjecting the derivative wherein the purine riboside has been halogenated at the 6-position and of which the hydroxyl groups are protected at the 3'-position and 5'-position thereof, to a deoxylation/Y-substitution reaction at the 2'-position thereof to introduce an intended substituent thereto at the 2'-position, and then subjecting it to a Z group-substitution reaction to the halogen atom at the 6-position thereof to introduce an intended substituent thereto at the 6-position, followed by further subjectiong it to a deprotection-deoxylation reaction at the 3'-position to deoxylate (remove) the hydroxyl group at the 3'-position thereof, the present inventors have made it possible to produce the above intended derivatives.

In producing the above nucleoside derivatives, one example is shown as a typical synthetic route in the following:

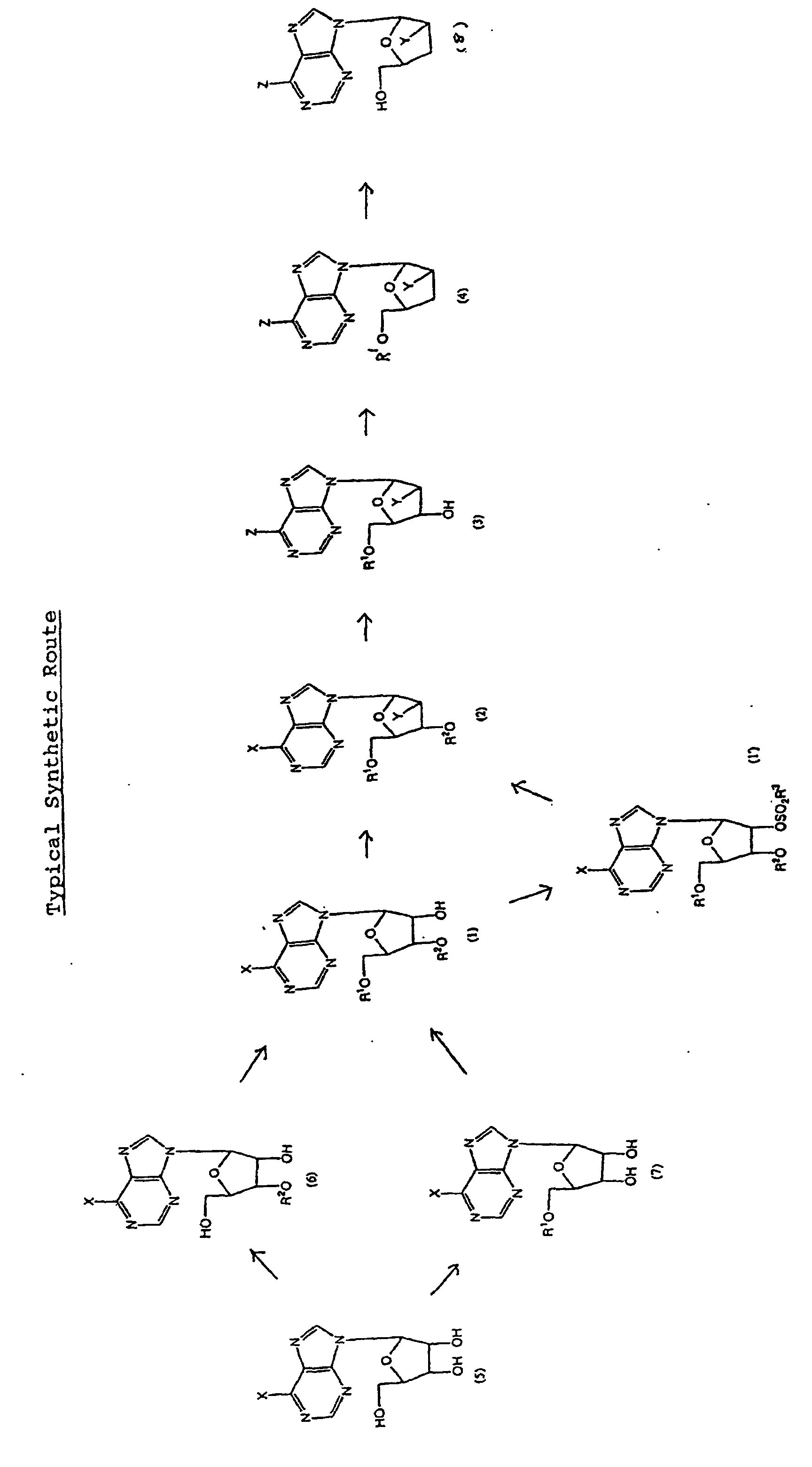

In the present invention, not only all the route shown in this synthetic route, of course, but also a step for production thereof comprising any one and every one of one single step contained therein (including a step for production of an intermediate), a novel intermediate and an use thereof, particularly an use thereof for production of the above-mentioned various nucleoside derivatives, etc. are contained, as illustrated hereinunder.

In those formulae, X represents a halogen atom, Y represents a substituent of any one of a fluorine atom, an azide group and a cyano group, Z represents any one of a hydrogen atom, an amino group, a hydroxyl group, an azide group, a substituent of a formula OR⁴, a substituent of a formula SR⁴ and a substituent of a formula NHR⁴, SO₂R³ represents a sulfonic acid-type leaving group, R¹ and R² are different from each other, each represents a protecting group for a hydroxyl group, and the both groups (R¹ and R²) can not be deprotected under the same condition; and
R⁴ represents a lower alkyl group (for example, carbons of 1 to 5) which may or may not have phenyl group(s) as substituent group(s).

In case of the sulfonic acid-type leaving group, preferably, R³ represents any one of a halogen atom, an aryl, alkyl or aralkyl group which may or may not have at least one substituent group, respectively and an alkylamino group which may or may not have at least one substituent group.

In the present invention, regarding the definitions and meanings on the signs used in the compounds represented by the general formula as shown before in the synthetic route, it shall be understood that the signs and the compounds designated by the same formula number are all common and same even though they are not individually described therein, and without any specified individual explanations therefor.

The present invention encompasses a novel process usable for producing the nucleoside derivatives including the novel intermediates and also the novel intermediates.

That is to say, the present invention encompasses a process as defined in claim 1. Preferred types of embodiment include:

1. A process for producing a nucleoside derivative represented by the following general formula (4), which comprises subjecting a derivative of purine riboside which is halogenated at the 6-position and of which the hydroxyl groups are protected at the 3'-position and the 5'-position thereof, to a deoxylation/Y-substitution reaction step at the 2'-position thereof;
subjecting it to a Z group-substitution reaction step for the halogen atom at the 6-position thereof; and subjecting it to a deprotection-deoxylation reaction step at the 3'-position thereof:

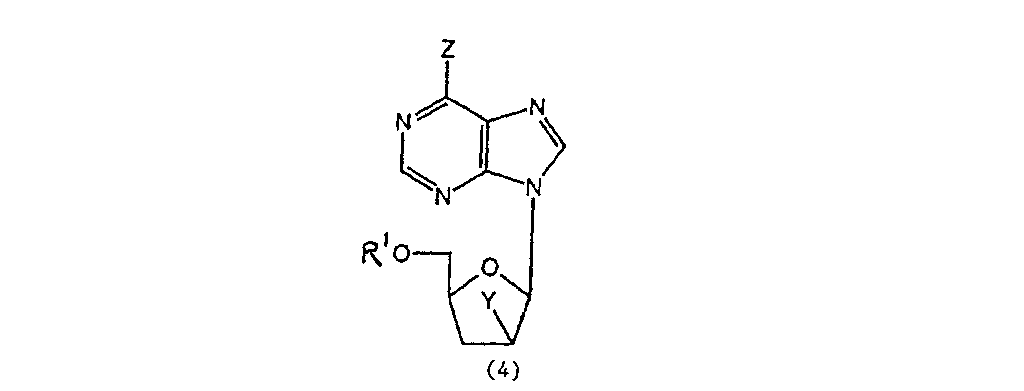

In the present invention, the step for Y-substitution reaction is a reaction step to introduce any one of the following given substituent groups which Y may represent after deoxylating (deoxidizing; -OH → -H) a hydroxyl group at the 2'-position thereof, and the Z group-substitution reaction step is a reaction step to substitute any one of the following given substituent groups which Z may represent for the halogen atom at the 6-position thereof.

In the above formula, Y represents a substituent of any one of a fluorine atom, an azide group and a cyano group;
Z represents any one of a hydrogen atom, an amino group, a hydroxyl group, an azide group, a
substituent of a formula OR⁴, a substituent of a formula SR⁴ and a substituent of a formula NHR⁴;
R¹ represents a protecting group (protective group) for a hydroxyl group; and
R⁴ represents a lower alkyl group (for example, a lower alkyl group having a carbon number of 1-5) which may or may not have substituent(s) being at least one phenyl group.

2. A process for producing a nucleoside derivative represented by the above described general formula (4), which comprises: subjecting a compound represented by the following general formula (3) obtained by
subjecting a compound represented by the following general formula (2): 6-halogen-substituted-9-(2-deoxy-2-substituted-β-D-arabinofuranosyl) purine derivative to a Z group-substitution reaction step for the halogen atom at the 6-position thereof and subjecting it to a deprotection (deblock) reaction step for the hydroxyl group at the 3'-position thereof, to a reaction step for a deoxylation (dehydroxylation; -OH→-H) of a hydroxyl group at the 3'-position thereof. 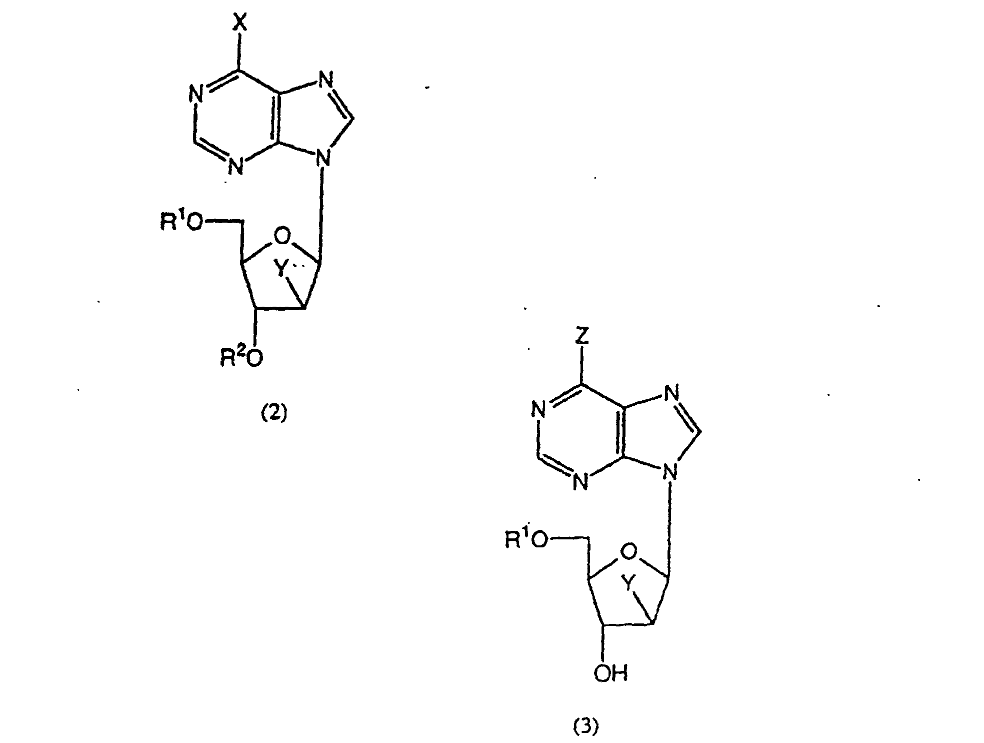 wherein the explanations on the Z group-substitution reaction step are the same as those described above, and X represents a halogen atom;
Y represents a substituent of any one of a fluorine atom, an azide group and a cyano group;
Z represents any one of a hydrogen atom, an amino group, a hydroxyl group, an azide group, a substituent of a formula OR⁴, a substituent of a formula SR⁴ and a substituent of a formula NHR⁴;
R¹ and R² are different from each other and each represents a protecting group for a hydroxyl group, of which both groups (R¹ and R²) can not be deprotected under the same condition (and at the same time); and
R⁴ represents a lower alkyl group wherein at least one hydrogen atom may or may not be replaced by phenyl group(s).

The nucleoside derivative (4) can be converted into the nucleoside derivative (8) of which the hydroxyl group has been deprotected at the 5'-position thereof, by further subjecting it to a deprotection reaction step for the hydroxyl group at the 5'-position thereof, if required, as described later, as well as the derivative obtained as described before.

Among the nucleoside derivatives in the present invention, the derivative (8) wherein the above Z represents a hydrogen atom, an amino group, a hydroxyl group, an azide group, a methylamino group, a methyloxy group, etc. is particularly preferable, since the derivative shows an effect useful for a pharmaceutical product such as an anti-viral activity.

On converting the above compound (2) into the compound (3), the 3'-position (R²) of the compound represented by the general formula (2) may be selectively deprotected (deblocked). In the case of the deprotection, the method and condition may be selected according to the varieties of the protecting group at the 3'-position thereof such that the 5'-positon thereof may not be deprotected.

In the compound represented by the general formula (2), in particular, preferably that in which X represents a chlorine atom and Y represents a fluorine atom, one embodiment of the process and/or method comprises a step of processing the compound to thereby substitute an amino group for the substituent group X, preferably processing it with a solution of ammonia in an alcohol (e.g., methanol, ethanol, propanol, etc.), or a step of processing the compound to thereby substitute a hydroxyl group for the substituent group X, preferably processing it with an aqueous solution of an alkali hydroxide (e.g., sodium hydroxide, potassium hydroxide, etc.), or a step of processing the compound to thereby substitute a hydrogen atom for the substituent group X, preferably processing it with hydrogen in the presence of a reduction catalyst (e.g., palladium-carbon, Raney nickel, etc.), or a step of processing the compound to thereby substitute an azide group for the substituent group X, preferably processing it with an alkali metal azide (e.g., sodium azide, lithium azide, etc.).

In introducing any one of a substituent of a formula OR⁴, a substituent of a formula SR⁴ and a substituent of a formula NHR⁴, it can be introduced easily in the process described later. In this case, R⁴ can represent a lower alkyl group (for example, a lower alkyl group having a carbon number of 1 to 5), which may or may not have substituent(s) being phenyl group(s).

3. The process as described above, which further comprises deprotecting (removing) the hydroxyl-protecting group at the 5'-position thereof to produce a compound represented by the following general formula (8):

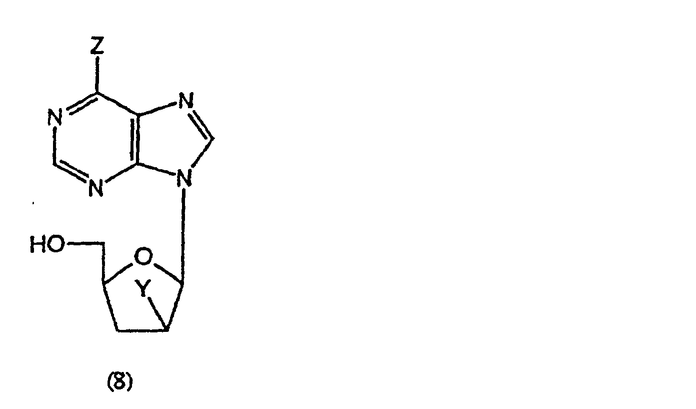

In the above formula, the explanations on the Y, Z and R⁴, are the same as those described above.

4. The process as described above, wherein the compound represented by the general formula (2) is a compound obtained by subjecting a compound represented by the following general formula (1) to a deoxylation/Y-substitution reaction step at the 2'-position thereof, or a compound obtained by subjecting a compound represented by the following general formula (1') to a removal of leaving group/Y-substitution reaction step at the 2'-position thereof: 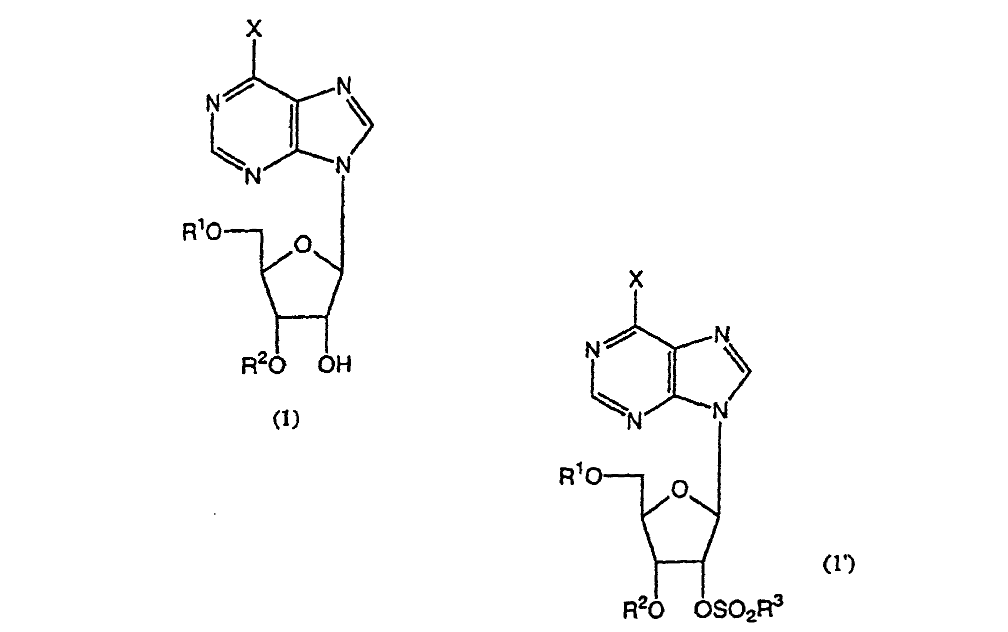 wherein the explanations on the step for Y-substitution reaction is the same as those described above, and in these steps and in the above formulae Y represents a substituent of any one of a fluorine atom, an azide group and a cyano group; X represents a halogen atom such as a chlorine atom, a bromine atom and an iodine atom; R¹ and R² are different from each other and each represents a protecting group for a hydroxyl group of which both substituent groups can not be deprotected under the same condition; and SO₂R³ represents a sulfonic acid-type leaving group.

Preferably, R³ represents a substituent of any one of a halogen atom, an aryl, alkyl or aralkyl group which may or may not have substituent(s) (for example, a halogen atom and the like) and also an alkylamino group which may or may not have substituent(s) (for example, a halogen atom and the like), as described before.

The deoxylation/Y-substitution reaction step at the 2'-position thereof is a reaction step to remove the hydroxyl group at the 2'-position thereof and introduce a fluorine atom, an azide group or a cyano group thereto, and preferably it may be reacted with an alkylaminosulfur · trifluoride reagent or a fluoroalkylamine reagent.

In the present invention, the removal of leaving group/Y-substitution reaction step at the 2'-position thereof is a reaction step to remove the sulfonic acid-type leaving group in the form of the O-sulfonic acid-type leaving group and substitute therefor a fluorine atom, an azide group or a cyano group which Y may represent therein. Preferably, it may be reacted with a reagent for substitution of a fluorine atom, an azide group or a cyano group therefor, for example, any one of an azide, an cyanide and a fluoride.

For the sulfonic acid-type leaving group, a leaving group of sulfonic acid-type which can be usually used as the leaving or removing group for a hydroxyl group in this field may be employed.

5. The process as described above, wherein the Z group-substitution reaction step at the 6-position thereof and the deprotection reaction step at the 3'-position thereof contain an alkaline treatment step.

6. The process as described above, wherein an alkaline material for the alkaline treatment is at least one selected from an ammonia and an alkaline water.

By treating it with an ammmonia or an alkaline water (alkaline aqueous solution), 9-(2-deoxy-2-substituted-β-D-arabinofuranosyl) adenine represented by the general formula (3) or a hypoxanthine derivative can be produced, and herein Z represents an amino group or a hydroxyl group.

In the case of the compound wherein R² is an acyl group, by treating (processing) it with an ammmonia, it can be converted into the derivative (3) wherein Z is an amino group.

7. The process as described above, wherein the Y-substitution reaction step is a fluorine atom-substitution reaction step, that is a reaction step for substituting a fluorine atom therefor, and in the above formula Y represents a fluorine atom.

8. The process as described above, wherein the Z group-substitution reaction step is an amino group-substitution reaction step, that is a reaction step for substituting an amino group therefor, and in the above formulae Z represents an amino group.

The invention may employ an intermediate represented by any one of the above-mentioned general formulae (1), (1'), (2) and (3).

In the above-mentioned formulae (1), (1'), (2) and (3), X represents a halogen atom such as a chlorine atom, a bromine atom and an iodine atom;
Y represents a substituent of any one of a fluorine atom, an azide group and a cyano group; Z represents any one of a hydrogen atom, an amino group, a hydroxyl group, an azide group, a substituent of a formula OR⁴, a substituent of a formula SR⁴ and a substituent of a formula NHR⁴; R¹ and R² are different from each other and each represents a protecting group for a hydroxyl group of which both substituents can not be deprotected under the same condition and at the same time; SO₂R³ represents a sulfonic acid-type leaving group; R⁴ represents a lower alkyl group which may or may not have substituent(s) being phenyl group(s); and R¹ does not contain an acyl group.

Particularly, the compound wherein R¹ represents an organic silyl group, for example, a trimethylsilyl group, a tert-butyl-dimethylsilyl group, and a trityl group which may or may not have substituent(s) such as paramethoxy group, di-paramethoxy group, tri-paramethoxy group, parachloro group, etc. and R² represents an acyl group having a carbon number of 1 to 12 is preferable for the above-mentioned intermediate.

More preferably the compound wherein Z represents an amino group or a hydroxyl group, and R¹ represents a protecting group for a hydroxyl group other than an acyl group, and further more preferably the compound wherein Z represents an amino group, and R¹ represents a trityl group which may or may not have substituent group(s) such as paramethoxy group, di-paramethoxy group, tri-paramethoxy group, parachloro group, etc. can be used for the above-mentioned intermediate, in particular as the intermediate (3).

The intermediate as described above may be one wherein in the general formulae, Z is an amino group or a hydroxyl group, R¹ is a trityl group which may or may not have substituent group(s), and R² is an acyl group.

In the process as described in any one of the above processes, the intermediate as described above may be used for the production of the above nucleoside derivative, or the intermediate as described above which is used for the production.

A process for producing such an intermediate may comprise at least one of the following steps,

### Step A:

A step for producing an intermediate represented by the above general formula (2), which comprises: (i) subjecting a compound represented by the above general formula (1) to a deoxylation/Y-substitution reaction step at the 2'-position thereof; or
(ii) subjecting a compound represented by the above general formula (1') to a removal of leaving group/Y-substitution reaction step at the 2'-position thereof;

### Step B:

A step for producing an intermediate represented by the above general formula (3), which comprises: subjecting a compound represented by the above general formula (2) to a Z group-substitution reaction step at the 6-position thereof and to a deprotection step at the 3'-position thereof, and

### Step C:

A step for producing an intermediate represented by the above general formula (1'), which comprises: subjecting a compound represented by the above general formula (1) to a reaction step for inserting a sulfonic acid-type leaving group thereinto.

In the above general formulae, X represents a halogen atom such as a chlorine atom, a bromine atom, an iodine atom, etc.; Y represents a substituent of any one of a fluorine atom, an azide group and a cyano group;
Z represents any one of a hydrogen atom, an amino group, a hydroxyl group, an azide group, a
substituent of a formula OR⁴, a substituent of a formula SR⁴ and a substituent of a formula NHR⁴;
SO₂R³ represents a sulfonic acid-type leaving group; and
R¹ and R² are different from each other and each represents a protecting group for a hydroxyl group of which both substiruent groups (R¹ and R²) can not be deprotected unde the same condition (at the same time); and
R⁴ represents a lower alkyl group which may have substituent(s) being phenyl group(s).

In the sulfonic acid-type leaving group, preferably R³ represents a substituent of any one of a halogen atom, an aryl, alkyl or aralkyl group which each may have optionally any substituent(s) (for example, a halogen atom and the like) and also an alkylamino group which may have optionally any substituent(s) (for example, a halogen atom and the like).

In the present invention, the reaction step for inserting the sulfonic acid-type leaving group thereinto is a reaction step to substitute for a hydrogen atom of the hydroxyl group the substituent group SO₂R³ which can be used as the sulfonic acid-type leaving group for a hydroxyl group. In the case of the reaction step for inserting the sulfonic acid-type leaving group thereinto in the above step C, it may be reacted with a sulfonyl halide or a sulfonic acid anhydride, or it may be reacted with a sulfuryl chloride and then with an amine or a halogen such as fluorine atom, etc.

In the process for producing the above nucleoside derivative, the process for producing the above intermediate may be used for the production thereof.

The process for producing an intermediate as described above may employ as a reagent used in the deoxylation/Y-substitution reaction step at the 2'-position, an alkylaminosulfur·trifluoride reagent or a fluoroalkylamine reagent; a reagent used in the reaction for removal of leaving group/Y-substitution reaction step is any one of an azide, a cyanide and a fluoride; and the reaction step for inserting a sulfonic acid-type leaving group comprises a reaction step for reacting the compound with a sulfonyl halide or a sulfonic acid anhydride, or a reaction step for reacting the compound with a sulfuryl chloride and then with an amine or a halogen such as fluorine.

### EMBODIMENTS OF INVENTION

Modes of carrying out the present invention are described below.

A compound represented by the general formula (1) noted above for use in the present invention is a 6-substituted-purine riboside, wherein the 6-position of inosine has been halogenated (chlorianted, brominated, iodized, etc.) and the hydroxyl groups at the 3'-position and the 5'-position thereof are protected with hydroxyl-protecting groups which are different from each other and can not be deprotected (deblocked) at the same time under the same condition.

For the protecting group (protective groups) for hydroxyl groups at the 3'-position and the 5'-position, for example, an acyl group having from 1 to 12 carbon atoms, an alkyl group having from 1 to 18 carbon atoms, an aralkyl group having from 7 to 19 carbon atoms, an organic silyl group and the like are exemplified. In order to produce FddA and the compounds related thereto of which the production is one object in the present invention, for the protecting group of the hydroxyl group at the 3'-position an acyl group such as acetyl group, benzoyl group, etc., and for the protecting group of the hydroxyl group at the 5'-position an aralky group such as a trityl group, a benzyl group, etc. are preferably employed. Particularly, for the protecting group of the 3'-hydroxyl group a benzoyl group is preferable, and for the protecting group of the 5'-hydroxyl group a trityl group which may have substituent(s) is preferable.

The compound represented by the above general formula (1) can be produced by using as the starting material the compound represented by the following general formula (5) of which a representative is 6-chloropurine riboside which can be synthesized by the known methods (for example, see B. R. Baker et al., J. Org. Chem., (22), page 954 (1957)), reacting the compound with a reagent for protection of a hydroxyl group under the presence of an activating agent to selectively protect the hydroxyl group at the 3'-position thereof and thereby to obtain a compound represented by the following general formula (6), and then reacting it under the presence of a base with a reagent for protection of a hydroxyl group other than the above reagent for protection for the 3'-position wherein the two protecting groups for the 3'-position and the 5'-position derived from the above 2 sorts of reagents can not be deprotected at the same time under the same condition. 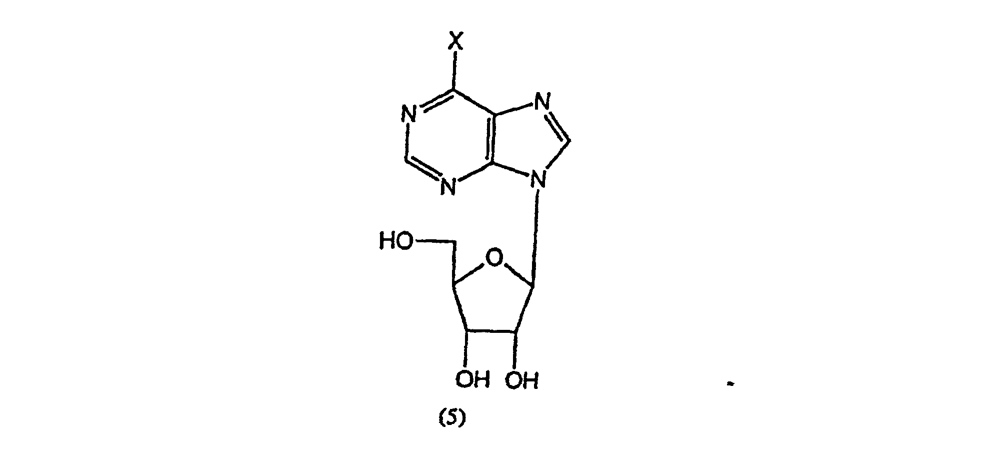 (In the formula, X represents a halogen atom such as chlorine atom, bromine atom, iodine atom, etc.) 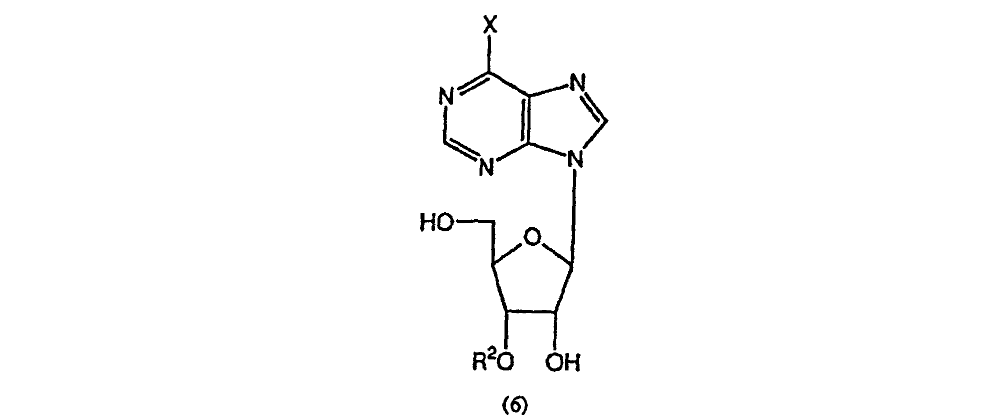 (In the formula, X represents a halogen atom in the same as described above and R² represents a protecting group for a hydroxyl group (hydroxyl-protecting group).)

In this case, a tin compound and a base may be employed for the activating agent. The tin compound is reacted with a compound represented by the general formula (5) to form an adduct, and the adduct is further reacted with a reagent for protection of a hydroxyl group to obtain a compound represented by the general formula (6) wherein the hydroxyl group at the 3'-position thereof is selectively protected.

When a base is used, a compound represented by the general formula (5) can be reacted under the presence of the base directly with a reagent for protection of a hydroxyl group to obtain a compound represented by the general formula (6) wherein the hydroxyl group at the 3'-position is selectively protected. In any cases, the mixture thereof with the compound wherein the hydroxyl group at the 2'-position is also protected and which is not a desired one, may be obtained depending on a combination of the reagent with the reaction condition. In such case, also when an acyl group is employed as the reagent for protection thereof, a transfer of acyl group as the protecting group from the 2'-position to the 3'-position thereof is effected from the mixture, and thus obtained product is crystallized to obtain exclusively the compound represented by the general formula (6) wherein the hydroxyl group at the 3'-position is selectively protected. The transfer of acyl group can be easily effected in a protic solvent such as an alcohol, for example, a methanol solution.

For the concrete examples of the tin compound, dialkyltin oxide such as di-n-butyltin oxide, dialkyltin dihalogenide such as dimethyltin dichloride are enumerated.

And, the base usable in the reaction includes, for example, hydroxylamine, ammonia and their salts; primary to quaternary amines and their salts; metal hydroxides such as barium hydroxide; metal alkoxides such as sodium methoxide and potassium methoxide; lithium-ammonia solution; ion exchange resins; carbonates such as potassium carbonate, sodium carbonate and sodium hydrogen carbonate; phosphates such as disodium hydrogen phosphate; acetates such as sodium acetate; and alkaline solutions of sodium hydroxide, lithium hydroxide or the like.

As the reagent that gives such a protective group (protecting group), for example, preferably used is any of an acylating agent, an alkylating agent, an aralkylating agent, an organic silylating agent and the like.

The acylating agent includes, for example, acid anhydrides such as acetic anhydride and benzoic anhydride, and acid halides such as acyl chloride and benzoyl chloride. The alkylating agent includes, for example, alkyl halides such as chloromethyl · methyl ether and allyl bromide. The aralkylating agent includes, for example, aralkyl halides such as benzyl bromide and triphenylmethyl · chloride. The organic silylating agent includes, for example, organic silyl halides such as trimethylsilyl chloride.

In the case that an ultimate intended product is a compound wherein a substituent at the 6-position in the nucleic acid base is an amino group such as FddA, an ammonia is used to convert the halogen atom into an amino group at the 6-position in the nucleic acid base.

Therefore, in view of the fact that the protecting group at the 3'-position thereof is removed at the same time, the hydroxyl group at the 3'-position thereof is preferably acylated, more specifically and more preferably acylated with an acetyl group or a benzoyl group. At that time, the hydroxyl group at the 5'-position thereof is preferably protected with a protecting group which can not be removed in the above described ammonia treatment, for example, a trityl group which may or may not have substituent group(s).

In such reaction for introducing a protecting group thereto at the 3'-position thereof, it may be reacted with the activating agent in an suitable solvent, preferably in an organic solvent such as ethyl acetate, toluene, methylene chloride, methanol, etc. It may be reacted with the activating agent at a temperature of the room temperature to the temperature heated for refluxing. After completion of the reaction, the reaction mixture can be reacted the reagent for protection thereof without isolating the adduct formed. In any cases, after completion of the reaction, the reaction mixture may be neutralized for the base therein, if required, and the intended compound can be isolated therefrom with a conventional extraction operation using an organic solvent such as ethyl acetate, toluene and methylene chloride. After completion of the reaction, the reaction mixture can be used for the next reaction without isolation of the product.

A compound represented by the general formula (1) can be obtained by reacting a compound represented by the general formula (6) which can be synthesized by the methods described above, with the reagent for protection thereof under the presence of the base to protect the hydroxyl group at the 5'-position thereof.

In order to achieve the object of the present invention, the reagent for the protection of a hydroxyl group used herein, is to be different from the reagent for protection thereof which may be used for the production of the compound represented by the general formula (6) from the compound represented by the general formula (5), and also is to be one which can introduce a protecting group thereinto so that the two protecting groups (R¹ and R²) as protected may not be removed at the same time under the same condition. As a result, when a compound represented by the general formula (3) is produced from a compound represented by the formula (2), the hydroxyl group at the 3'-position thereof can be protected selectively.

As the protecting group for hydroxyl group at the 5'-position thereof, for example, an acylating agent, an alkylating agent, an aralkylating agent, an organic silylating agent, etc. are enumerated. The acylating agent includes, for example, acid anhydrides such as acetic anhydride and benzoic anhydride, and acid halides such as acyl chloride and benzoyl chloride. The alkylating agent includes, for example, alkyl halides such as chloromethyl· methyl ether and allyl bromide. The aralkylating agent includes, for example, aralkyl halides such as benzyl bromide and triphenylmethyl · chloride. The organic silylating agent includes, for example, organic silyl halides such as trimethylsilyl chloride.

In the case that an ammonia is used for converting the halogen atom into the amino group at the 6-position in the nucleic acid base for production of a FddA which is an ultimate intended product and the like, the hydroxyl group at the 3'-position thereof is preferably protected with an acyl group in view of removing possibly the protecting group at the 3'-position at the same time, as described above.

Accordingly, for the protecting group for the hydroxyl group at the 5'-position thereof, specifically the protecting group other than an acyl group such as an acetyl group or a benzoyl group, for example, a trityl group which may or may not have substituent group(s) is preferable.

And, the base usable in the reaction wherein a compound represented by the formula (1) is produced from a compound represented by the formula (6) includes, for example, hydroxylamine, ammonia and their salts; primary to quaternary amines and their salts; metal hydroxides such as barium hydroxide; metal alkoxides such as sodium methoxide and potassium methoxide; lithium-ammonia solution; ion exchange resins; carbonates such as potassium carbonate, sodium carbonate and sodium hydrogen carbonate; phosphates such as disodium hydrogen phosphate; acetates such as sodium acetate; and alkaline solutions such as sodium hydroxide, lithium hydroxide and the like.

The reaction can be carried out in a suitable solvent, preferably in an organic solvent such as ethyl acetate, toluene, methylene chloride, methanol, etc. After completion of the reaction, the reaction mixture may be neutralized for the base therein, if required, and the intended compound can be isolated therefrom with a conventional extraction operation using an organic solvent such as ethyl acetate, toluene, methylene chloride, etc. After completion of the reaction, the reaction mixture may be used for the next reaction step without isolation of the product.

In these reactions, for example, in the reaction for protection of a hydroxyl group with a comparatively sterically bulky protecting group such as triphenylmethyl (trityl) group, tert-butyl-dimethylsilyl, etc., the reaction is caused selectively for the hydroxyl group at the 5'-position thereof corresponding to a hydroxyl group of the primary alcohol under an usual reaction condition. In the other reagents for protection thereof, also the reaction can be raised selectively for the hydroxyl group at the 5'-position thereof by keeping the reaction temperature low.

As understood from the explanations described before, an acyl group such as an acetyl group, a benzoyl group, etc. used as the protecting group for the hydroxyl group at the 3'-position thereof, and an aralkyl group such as trityl group which may have substituent(s), a benzyl group, etc. used as the protecting group for the hydroxyl group at the 5'-position thereof, are preferably employed in combination.

Now, a compound represented by the general formula (1) may be produced by reacting a compound represented by the general formula (5) with a reagent for the protection of a hydroxyl group under the presence of the base to produce a compound represented by the following general formula (7): 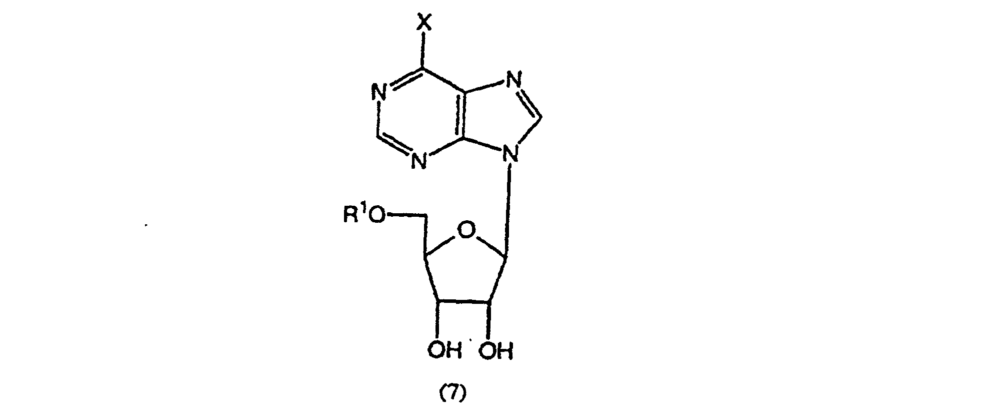 wherein X represents a halogen atom which is in the same as those described above, and R¹ represents a protecting group for a hydroxyl group,
and reacting the same under the presence of the activating agent with the reagent for protection of a hydroxyl group different from the above reagent for the R¹ group.

A compound represented by the general formula (1) obtained in the processes described above is an useful and important intermediate which can lead to a FddA corresponding to one compound of the objective compounds desired in the present invention and the other nucleic acid derivatives.

In order to a 6-substituted- 9-(2-deoxy-2-substituted-β-D-arabinofuranosyl) purine derivative by substituting a fluorine atom, an azide group or a cyano group for the hydroxyl group at the 2'-position of a compound represented by the general formula (1) in the present invention, a compound represented by the general formula (1) may be subjected to a reaction step for inserting the sulfonic acid-type leaving group (SO₂R³) thereinto, for example, it may be reacted with a sulfonyl halide or a sulfonic acid anhydride, or it may be reacted with sulfuryl chloride and then with an amine or a halogen, to produce a compound represented by the general formula (1') wherein the explanations on the groups X, R¹, R² and R³ are the same as those described above, and thus obtained compound may be reacted with an azide, a cyanide or a fluoride compound.

In this case, particularly the compound represented by the general formula (1) wherein R¹ is a trityl group which may have substituemt(s) and R² is an acyl group having a carbon number of 1 to 12 is preferable therefor.

The sulfonyl halide includes, for example, arylsulfonyl halides such as paratoluenesulfonyl chloride and paranitrobenzenesulfonyl · chloride; alkylsulfonyl · halides such as methane · sulfonyl chloride; aralkylsulfonyl · halides such as benzylsulfonyl · chloride; and halogenoalkylsulfonyl · halides such as trifluoromethanesulfonyl · chloride.

The sulfonic acid anhydride includes, for example, arylsulfonic acid anhydrides such as paratoluenesulfonic acid anhydride and paranitrobenzenesulfonic acid anhydride; alkylsulfonic acid anhydrides such as methanesulfonic acid anhydride; aralkylsulfonic acid anhydrides such as benzylsulfonic acid anhydride; and halogenoalkylsulfonic acid anhydrides such as trifluoromethanesulfonic acid anhydride. The amine includes, for example, imidazole, etc. The halogen includes, for example, fluorine, etc.

In view of the above, for the group R³ in a sulfonic acid-type leaving group (SO₂R³), a halogen atom such as a chlorine atom, an aryl (having from 6 to 10 carbon atoms, such as phenyl group), alkyl (having from 1 to 5 carbon atoms) or aralkyl (having from 7 to 19 carbon atoms, such as benzyl group) group which may or may not have substituent group(s) (for example, a halogen atom such as chlorine atom, an alkyl group having from 1 to 5 carbon atoms, a nitro group, an alkyloxy group of which the alkyl moiety has from 1 to 5 carbon atoms, and the like), and also an alkylamino group (having from 1 to 6 carbon atoms) which may or may not have substituent group (s) (for example, a halogen atom such as cholorine atom, an alkyl group having from 1 to 5 carbon atoms, a nitro group, an alkyloxy group of which the alkyl moiety has from 1 to 5 carbon atoms, and the like) may be preferably employed. More preferably, a chlorosulfonyl group, a fluorosulfonyl group, an imidazolesulfonyl group, a trifluoromethanesulfonyl group, a methanesulfonyl group, an arylsulfonyl group such as a paratoluenesulfonyl group, paranitrobenzenesulfonyl group and benzenesulfonyl group, etc., and the like are enumerated.

The reaction can be carried out in an suitable solvent, preferably in an organic solvent such as ethyl acetate, toluene, methylene chloride, etc. And, the reaction may be carried out under the presence of a basic catalyst such as pyridine; dimethylaminopyridine, triethylamine, etc. After completion of the reaction, the reaction mixture may be neutralized for the base therein, if required, and the intended compound can be isolated therefrom with a conventional extraction operation using an organic solvent such as ethyl acetate, toluene and methylene chloride, etc. After completion of the reaction, the reaction mixture may be used for the next reaction step without isolation of the product.

A compounds represented by the general formula (2) can be obtained by reacting a compound represented by the general formula (1') with an azide, a cyanide or a fluoride. The azide includes, for example, alkali metal azides such as sodium azide and lithium azide, ammonium azide, trimethylsilyl azide and the like. The cyanide includes, for example, alkali metal cyanides such as sodium cyanide and lithium cyanide. The fluoride includes, for example, hydrogen fluoride: alkali metal fluorides such as lithium fluoride, potassium fluoride and cesium fluoride; alkylammonium fluorides such as tetrabutylammonium fluoride, pyridinium·polyhydrogenfluoride and triethylamine trihydrofluoride; alkylaminosulfur·trifluorides such as diethylaminosulfur·trifluoride and morpholinosulfur·trifluoride; and fluoroalkylamines such as Yarovenko reagent and Ishikawa reagent.

The reaction can be carried out in an suitable solvent, preferably in an organic solvent such as ethyl acetate, toluene, methylene chloride, etc. The reaction may be carried out under the presence of a basic catalyst such as pyridine, dimethylaminopyridine, triethylamine, etc. After completion of the reaction, the reaction mixture may be neutralized for the basic catalyst therein, if required, and the intended compound can be isolated therefrom with a conventional extraction operation using an organic solvent such as ethyl acetate, toluene and methylene chloride.

A compound represented by the general formula (2) obtained in the process described above is an useful and important intermediate which can lead to a FddA corresponding to one of the object compound desired in the present invention and the other nucleic acid derivatives.

A compound represented by the formula (2) noted above, wherein Y is a fluorine atom, can be obtained also by reacting a compound represented by the formula (1) with a fluoride compound. The fluoride for this includes, for example, alkylaminosulfur·trifluorides such as diethylaminosulfur·trifluoride and morpholinosulfur·trifluoride. This reaction may be effected in a suitable solvent. For this, preferably used is an organic solvent such as ethyl acetate, toluene and methylene chloride. The reaction may be carried out under the presence of a basic catalyst such as pyridine, dimethylaminopyridine, triethylamine, etc.

A compound represented by the general formula (3) can be obtained by deprotecting (removing) selectively the protecting group of the protected hydroxyl group at the 3'-psition of the compound represented by the general formula (2). In the deprotection thereof, a method in which the protected hydroxyl group at the 5'-position thereof is not deprotected is employed according to a kind of the protecting group at the 3'-position thereof. For example, in case that the R² is an alkyl group having from 1 to 18 carbon atoms or an aralkyl group having from 7 to 19, it may be deprotected by reduction, hydrolysis, etc. thereof in a suitable solvent. In case that the R² is an acyl group, it may be deprotected by reduction thereof in a suitable solvent. In case that the R² is an organic silyl group, it may be deprotected by reduction thereof in a suitable solvent or by the use of an agent for deprotecting (removing) a silyl group of the sililated hydroxyl group.

Particularly, a compound represented by the general formula (2) wherein the R² is an acyl group, may be converted to a compound represented by the general formula (3) wherein Z represents an amino group by processing it with an ammonia. In this case, an amination at the 6-position thereof and a deprotection at the 3'-position thereof are carried out at the same time. It may be reacted in an aqueous solution containing ammonia at an equivalent or more (excess) amount thereto, or a solution of a suitable organic solvent such as methanol, ethyl acetate, etc. Preferably, it may be reacted in a solution of methanol saturated with ammonia. The reaction may be carried out at a temperature selected optionally from the temperature of ice to the temperature heated for refluxing. When it may be reacted at a temperature higher than the room temperature, the reaction may be preferably carried out in a sealed tube to avoid a loss of the ammonia outside of the system. After completion of the reaction, the ammonia and solvent are distilled off and the residue is purified to obtain an intended product.

To produce a derivative (3) wherein Z represents an amino group, it may be processed with ammonia as dissolved in an alcohol such as methanol under pressure. To produce a derivative (3) wherein Z represents a hydroxyl group, it may be processed with an aqueous solution of an alkali hydroxide such as sodium hydroxide and potassium hydroxide. And, to produce a derivative (3) wherein Z represents a hydrogen atom, it may be processed with hydrogen under the presence of a reduction catalyst such as palladium-carbon.

To produce a derivative (3) wherein Z represents an azide group, it may be processed with an alkali metal azide, such as sodium azide or lithium azide, in a solvent capable of dissolving the metal azide, such as dimethylformamide. To produce a derivative (3) wherein Z represents OR⁴ or SR⁴, it may be processed with a corresponding alkyl alcohol or alkyl thiol having been activated with an alkali metal halide such as sodium halide.

To produce a derivative (3) wherein Z represents NHR⁴, it may be processed with an alkylamine (corresponding to the intended substituent group, such as methylamine), preferably in an inert solvent such as dimethylformamide.

In the above formula, R⁴ indicates a lower alkyl group which may have optionally phenyl group(s) as the substituent group(s), and the lower alkyl group includes, for example, an alkyl group of carbons: 1 to 5, such as methyl group, ethyl group, propyl group, butyl group, benzyl group and the like.

A compound represented by the general formula (4) in the present invention may be obtained with ease by deoxylating (dehydroxylating:-OH→-H) the hydroxyl group at the 3'-position of a compound represented by the formula (3). For the process of deoxylation, there is a process for forming a thionocarbonyl ester thereof such as methylthionocarbonyl ester, phenyloxythionocarbonyl ester, etc., and reducing it with a radical reaction reagent, such as tri-n-butyl tin hydride, tris(trimethylsilyl) silane, diphenylsilane, diphenylmethylsilane or the like under the presence of a radical reaction initiator such as azobisisobutyronitrile, etc.

In the reaction, first the corresponding thionocarbonyl · chloride may be reacted therewith or carbon · disulfide, and alkyl alcohol or alkyl thiol are reacted therewith to form a thionocarbonyl ester. It may be reacted in a base at an equivalent amount thereto. It may be reacted in a suitable solvent, preferably an organic solvent such as ethyl acetate, toluene, methylene chloride, acetonitrile, etc. The reaction may be carried out, for example, at the temperature of - 80°C to the temperature heated for refluxing the solvent employed. After completion of the reaction, the reaction mixture is neutralized for the base, if required, and the thionocarbonyl ester can be isolated by using an ordinal extraction operation with the use of an organic solvent such as ethyl acetate, toluene, methylene chloride, etc. After completion of the reaction, the reaction mixture per se can be employed for the next reaction step without isolating the thionocarbonyl ester.

In the next reduction reaction, it may be reacted using a radical reaction reagent at an equivalent or more (excess) amount thereto, preferably in an organic solvent such as ethyl acetate, toluene, methylene chloride, acetonitrile, etc., or it may be reacted in the radical reaction reagent per se at an amount used for the solvent. The reaction may be carried out at the temperature of the room temperature to the temperature heated for refluxing the solvent. The radical reaction initiator can be employed in an equivalent or more amount thereto; and it may be employed usually sufficient in an amount used for catalyst. After completion of the reaction, from the reaction mixture the product can be isolated with usual extraction with the use of an organic solvent such as ethyl acetate, toluene, methylene chloride, etc.

A compound represented by the general formula (4) obtained in the above operations and steps and wherein Y is a fluorine atom and Z is an amino group, can be easily converted into a FddA which is one of the intended compound in the present invention by deprotecting the R¹ of the protecting group for the hydroxyl group at the 5'-position thereof. For example, the compound wherein the protecting group for the hydoroxyl group at the 5'-position thereof is a trityl group which may have substituent(s), may be processed with an acid such as acetic acid to easily deprotect it (remove the protecting group). For the other derivatives, it can be effected by subjecting it to a process for the deprotection thereof which is known as it is. For example, in the case of the compound wherein the R¹ is an acyl group such as benzoyl group, etc., a nucleoside derivative represented by the formula (8) may be produced by processing the compound with an alkaline material such as sodium hydroxide, potassium hydroxide, etc. In the case of the compound wherein R¹ is an alkyl group such as methoxymethyl group, allyl group, etc., it may be also produced by processing it with an acid such as hydrochloric acid, acetic acid, etc. In the case of the compound wherein the R¹ is an aralkyl group such as benzyl, triphenylmethyl, etc. it may be also produced by processing it with hydrogen in the presence of a reduction catalyst such as palladium-carbon and Raney nickel, or processing it with an acid such as acetic acid, if required.

### PREFERRED EMBODIMENTS

Now, the present invention is described in detail with reference to the following Examples.

### (Example 1)

### Synthesis of 3'-O-benzoyl-6-chloropurine riboside; Part-1

6-Chloropurine riboside (14.34 g, 50 mmol) and di-n-butyltin oxide (12.45 g, 50 mmol) are suspended in methanol (500 ml), and the mixture is heated under refluxing for 1 hour. After cooling, triethylamine (34.8 ml, 250 mmol) is added thereto and then benzoyl chloride (29.0 ml, 250 mmol) is added dropwise thereto while stirring. The mixture is stirred for 30 minutes at room temperature, and then an insoluble material is removed by filtration. Thus obtained filtrate is concentrated. To the residue, ether (400 ml) and water (250 ml) are added, and then the water layer is washed twice with ether (100 ml). The organic layers are combined together, and washed with water (100 ml). The ether layer is subjected to the dry column (6.5×35cm), and a elution with dichloromethane (1 liter), is carried out. And then, a elution with dichloromethane (4 liters) containing 0 to 10% of ethanol is carried out.

Fractions containing a mixture of 2'-O-benzoyl derivative and 3'-O-benzoyl derivative are collected, and the solvent is distilled off therefrom. The resulting residue is crystallized with methanol to obtain 3'-O-benzoyl derivative in a white crystalline form (yield: 12.67 g, FW: 390.8, 32.5 mmol, 65 %). Melting point: 174-177°C.
¹H-NMR (CDCl₃) δ : 8.97, 8.91 (each 1H, s, H2, H8), 7.5-8.1 (5H, Bz), 6.16 (1H, d, J=6.5Hz, H1'), 5.95 (1H, m, 2'-OH), 5.55 (1H, dd, J=6.5, J=2.4Hz, H3'), 5.28 (1H, brs, 5'-OH), 5.00 (1H, m, H2'), 4.33 (1H, m, H4'), 3.72 (2H, t, J=2.8, H5').

### (Example 2)

### Synthesis of 3'-O-benzoyl-6-Chloropurine riboside; Part-2

6-Chloropurine riboside (34.93 g, 122 mmol) and di-n-butyltin oxide (30.35 g, 122 mmol) are suspended in methanol (1200 ml), and the mixture is heated under refluxing for 1 hour. After cooling, triethylamine (37.02 g, 366 mmol) is added thereto and then benzoyl chloride (51.43 g, 366 mmol) is added dropwise thereto while stirring. The mixture is stirred for 2 hours at room temperature, and then triethylamine (12.34 g, 122 mmol) is added thereto, and then benzoyl chloride (17.12 g, 122 mmol) is added dropwise thereto while stirring.

The mixture is further stirred for 2 hours at room temperature and then an insoluble material is removed by filtration. Thus obtained filtrate is concentrated.

To the residue, ether (950 ml) and water (600 ml) are added, and then thus formed crystals are filtrated. Thus obtained crystals are dried under reduced pressure at 45°C. The crystals are suspended in water (260 ml) and then stirred for 50 minutes at room temperature. The crystals are filtrated, washed with water and dried under reduced pressure at 45°C to obtain 3'-O-benzoyl derivative in a white crystalline form (yield: 29.5 g; FW: 390.8, 75.5 mmol, 62 %).

### (Example 3)

### Synthesis of 5'-O-trityl-3'-O-benzoyl-6-chloropurine riboside

3'-O-benzoyl-6-chloropurine riboside (10.72 g, 25 mmol) is dissolved in dried dimethylformamide (220 ml), and thereafter 11.0 ml (82.5 mmol) of triethylamine (FW: 101.2, d=0.726) and 1.01 g (8.25 mmol) of 4-dimethylaminopyridine (FW: 122.2) are added thereto. After that, 23.00 g (82.5 mmol) of trityl chloride (FW: 278.8) is added thereto. The mixture is stirred overnight at 50°C. After cooling, water (10 ml) is added thereto. The solvent is distilled off and the resulting residue is dissolved in chloroform (300 ml) and water (150 ml). The organic layer is washed with water (150 ml), dried with magnesium sulfate and filtrated. The resulting solution is subjected to a column (3.5×50 cm) chromatography with the use of silica gel, and an elution with dichloromethane (1000 ml) and next 0 to 2.5 % ethanol/dichloromethane solution (4000 ml) is carried out. The solvent is distilled off, and the residue is crystallized with methanol to obtain a white crystals (yield: 11.21 g; FW: 633.1, 17.7 mmol, 71%). Melting point: 102-106 °C.
¹H-NMR(CDCl₃) δ : 8.69, 8.38 (each 1H, s, H2, H8), 7.2-8.05(ca 20H, Bz, Tr), 6.18 (1H, d, J=6.1Hz, H1'), 5.74 (1H, dd, J=5.5, J=2.5, H3'), 5.29 (1H, m, H2'), 4.59 (1H, m, H4'), 3.95 (1H, d, J=4.5Hz, 2'-OH), 3.53 (2H, t, J=3.6Hz, H5').

### (Example 4)

### Synthesis of 6-chloro-9-(5-O-trityl-3-O-benzoyl-2-deoxy-2-fluoro-β-D-arabinofuranosyl)-9H-purine; Part-1

5'-O-trityl-3'-O-benzoyl-6-chloropurine riboside (4.76 g, 7.5 mmol) is dissolved in dried dichloromethane (100 ml) and then pyridine (3.6 ml, 44.5 mmol) is added thereto. The mixture solution is cooled with ice and then diethylaminosulfur·trifluoride (DAST, 2.25 ml, 17 mmol) is added dropwise thereto while stirring. The mixture is heated to a room temperature and heated further under refluxing for 5 hours. After cooling, the mixture is added dropwise to5% sodium hydrogen carbonate aqueous solution (500 ml) under vigorously stirring and then the mixture is further stirred for 20 minutes. The mixture is put into a separating funnel and is shaken well, and then the organic layer is obtained. The water layer is washed with chloroform (100 ml). The organic layers are combined together, washed with water (200 ml), dried with magnesium sulfate and filtrated. The solvent is distilled off. The residue is azeotropicaly distilled with toluene until the odor or smell of pyridine is lost and then dissolved in benzene (50 ml). The solution is subjected to a silica gel column (3.5×50 cm) chromatography. An elution with 0 to 12.5 % ethyl acetate/benzene solution (4000 ml) is carried out. The fractions of the product are collected, and the solvent is distilled off to obtain a caramel yielding 3.80 g (FW: 635.1, 5.99 mmol, 80 %).
¹H-NMR(CDCl₃) δ : 8.76(1H, s, H2), 8.36(1H, d, J=3.0Hz, H8), 7.2-8.1(ca20H, Bz, Tr), 6.66(1H, dd, J=21.7, J=2.7Hz, H1'), 5.70(1H, dd, J=17.0, J=3.0Hz, H3'), 5.28(1H, ddd, J=50.0, J=3.0, J=0.8Hz, H2'), 4.42(1H, m, H4'), 3.62(1H, dd, J=10.4, J=5.2Hz, H5'a), 3.54(1H, dd, J=10.4, J=4.1Hz, H5'b).

### (Example 5)

### Synthesis of 5'-O-trityl-3'-O-benzoyl-2'-O-sulfurylimidazolyl-6-chloropurine riboside

5'-O-trityl-3'-O-benzoyl-6-chloropurine riboside (2.0 g, 3.1 mmol) is dissolved in dichloromethane (15.5 ml) and cooled to -35°C. Pyridine (0.45 ml, 5.57 mmol) and sulfuryl chloride (0.30 ml, 3.72 mmol) are added thereto, and then stirred for 30 minutes. Imidazole (0.96 g, 13.9 mmol) is added thereto. The mixture is heated to a room temperature and stirred overnight. Water (10 ml) is added thereto to prepare separated layers therein and the water layer is washed with dichloromethane (10 ml). The organic layers are combined together, dried with sodium sulfate and filtrated. The solvent is distilled off and the residue is purified on a silica gel column chromatography to obtain an oil yielding 2.28 g (FW: 763.22, 2.99 mmol, 96 %).
¹H-NMR(CDCl₃) δ : 8.63(1H, s, H2), 8.30(1H, s, H8), 7.2-8.1(ca20H, Bz, Tr), 7.71(1H, m, imidazole), 6.99(1H, m, imidazole), 6.87(1H, m, imidazole), 6.34(1H, d, J=5.0Hz, H1'), 6.22(1H, t, J=5.2, H2'), 5.79(1H, t, J=5.0, H3'), 4.53(1H, m, H4'), 3.62(1H, dd, J=2.9, J=11.0Hz, H5'a), 3.52(1H, dd, J=3.4, J=11.0Hz, H5'b).

### (Example 6)

### Synthesis of 6-chloro-9-(5-O-trityl-3-O-benzoyl-2-deoxy-2-fluoro-β-D-arabinofuranosyl)-9H-purine; Part-2

5'-O-trityl-3'-O-benzoyl-2'-O-sulfurylimidazolyl-6-chloropurine riboside (377.1 mg, 0.494 mmol) is dissolved in toluene (2.4 ml) and triethylamine·trihydrofluoride (0.49 ml, 3.00 mmol) is added thereto, and the resulting mixture is stirred at 70°C overnight. After cooling, to the mixture ethyl acetate and saturated sodium hydrogen carbonate aqueous solution are added to prepare separated layers therein.

The organic layer is washed with ethyl acetate. The organic layers are combined together, dried with sodium sulfate and filtrated. The solvent is distilled off.

The residue is dissolved in acetonitrile and analyzed with a liquid chromatography to obtain a desired product in a yield of 81 %.

### (Example 7)

### Synthesis of 5'-O-trityl-3'-O-benzoyl-2'-O-trifluoromethanesulfonyl-6-chloropurine riboside

5'-O-trityl-3'-O-benzoyl-6-chloropurine ribside (1.31 g, 2.0 mmol) is dissolved in toluene (20 ml) and to the mixture 4-dimethylaminopyridine (293 mg, 2.4 mmol) and trifluoromethanesulfonyl chloride (404 mg, 2.4 mmol) are added, and then stirred overnight at room temperature. Water (10 ml) is added thereto to prepare separated layers therein, and then the organic layer is washed with saturated sodium hydrogen carbonate aqueous solution, saturated ammonium chloride aqueous solution and saturated sodium chloride aqueous solution, in this order. The organic layer is dried with magnesium sulfate and filtrated. The solvent is distilled off to obtain a foamed solid, which is almost a single substance in the analysis with HPLC, yielding 1.35 g and thus obtained solid material is directly used for the next reaction.

### (Example 8)

### Synthesis of 6-chloro-9-(5-O-trityl-3-O-benzoyl-2-deoxy-2-fluoro-β-D-arabinofuranosyl)-9H-purine; Part-3

5'-O-trityl-3'-O-benzoyl-2'-O-trifluoromethanesulfonyl-6-chloropurine riboside (0.675g, about 1.0 mmol) is dissolved in etyl acetate (10 ml) and to the mixture triethylamine (304 mg, 3.0 mmol) and triethylamine·trihydrofluoride (967 mg, 6.0 mmol) are added. The mixture is heated under refluxing overnight while stirring. After cooling, all the mixture is dissolved in methanol and obtain a desired product in a yield of 88 % in the analysis by means of a liquid chromatography.

### (Example 9)

### Synthesis of 9-(5-O-trityl-2-deoxy-2-fluoro-β-D-arabinofuranosyl) adenine

6-Chloro-9-(5-O-trityl-3-O-benzoyl-2-deoxy-2-fluoro-β-D-arabinofuranosyl)-9H-purine (3.15 g, 4.98 mmol) is dissolved in methanol/ammonia (100 ml; saturated at 0 °C) and the mixture is allowed to stand for 2 days at 100 °C in a sealed tube. After cooling, the solvent is carefully distilled off and the residue is dissolved in chloroform (100 ml). An insoluble material is removed by filtration, and the filtrate is subjected to a silica gel column (3.5×50 cm) chromatography, which is eluted with 3 to 10 % ethanol/dichloromethane solution (4000 ml). The fractions of the product are collected and the solution is concentrated to obtain white crystals (1.87 g, 3.66 mmol, 73 %). Melting point: 210.5-212.5 °C.

### (Example 10)

### Synthesis of 9-(5-O-trityl-3-phenoxythiocarbonyloxy-2-deoxy-2-fluoro-β-D-arabinofuranosyl) adenine

4-Dimethylaminopyridine (273 mg) is dissolved in dehydrated acetonitrile (10 ml), and 9-(5-O-trityl-2-deoxy-2-fluoro-β-D-arabinofuranosyl) adenine (419 mg, 0.82 mmol) is suspended in this silution.

To the mixture under stirring phenoxythiocarbonyl chloride (0.22 ml) is added and the mixture is stirred at room temperature for 3 hours. The reaction solution is concentrated and the residue is dissolved in chloroform (20 ml) and subjected to a silica gel column (2.5X32cm) chromatography, which is eluted with 0 to 10 % ethanol/chloroform solution (1200 ml). The fractions of the product are collected and the solvent is distilled off to obtain a caramel in a yield of 490 mg (FW: 615.64, 0.80 mmol, 97 %).

### (Example 11)

### Synthesis of 9-(5-O-trityl-2,3-dideoxy-2-fluoro-β-D-threo-pentofuranosyl) adenine; Part-1

9-(5-O-trityl-3-phenoxythiocarbonyloxy-2-deoxy-2-fluoro-β-D-arabinofuranosyl) adenine (386 mg, 0.63 mmol) is suspended in dried toluene (4 ml), and to the mixture azobisisobutylonitrile (60 mg) and tris(trimethylsilyl)silane (0.6 ml) are added. The resulting mixture is stirred at 100 °C for 30 minutes under a current of nitrogen. After cooling, thus precipitated crystals are obtained on a filter paper, washed with toluene and dried. Thus precipitated crystals are found to be almost a single substance in the analysis with a thin layer chromatography, yielding 229 mg (0.46 mmol, 73 %). Melting point: 226-229 °C.
¹H-NMR(CDCl₃)δ: 8.36(1H, s, H2), 8.07(1H, d, J=2.6Hz, H8), 7.1-7.6(ca, 20H, Tr), 6.33(1H, dd, J=17, J=3.0Hz, H1'), 5.70(2H, brs, NH₂), 5.22(1H, m, J=43.6Hz, H2'), 4.4(1H, m, H4'), 3.46(1H, dd, J=8.2, J=5.9Hz, H5'a), 3.26(1H, dd, J=8.2, J=3.2Hz, H5'b), 2.2-2.65(2H, m, H3').

### (Example 12)

### Synthesis of 9-(5-O-trityl-2,3-dideoxy-2-fluoro-β-D-threo-pentofuranosyl) adenine; Part-2

9-(5-O-trityl-3-phenoxythiocarbonyloxy-2-deoxy-2-fluoro-β-D-arabinofuranosyl)adenine (386 mg, 0.63 mmol) is suspended in dried dioxane (8 ml), and to the mixture azobisisobutylonitrile (120 mg) and diphenyl silane (1.2 ml) are added. The resulting mixture is stirred at 115 °C for 2.5 hours under a current of nitrogen. After cooling, the solution is concentrated and the residue is dissolved in dichloromethane (10 ml) and subjected to a silica gel column (2.1×40 cm) chromatography, which is eluted with 0 to 10 % ethanol/dichloromethane solution (1100 ml). The fractions of the product are collected and the solvent is distilled off. The residue is crystallized with benzene to obtain white crystals (240 mg, 77 %). Also, a starting material (36 mg, 9.3 %) is recovered.

### (Example 13)

### Synthesis of 9- (5-O-trityl-2,3-dideoxy-2-fluoro-β-D-threo-pentofuranosyl) adenine; Part-3

9-(5-O-trityl-3-phenoxythiocarbonyloxy-2-deoxy-2-fluoro-β-D-arabinofuranosyl) adenine (386 mg, 0.63 mmol) is suspended in dried toluene (8 ml), and to the mixture azobisisobutylonitrile (120 mg) and diphenyl silane (1.2 ml) are added. The resulting mixture is stirred at 115 °C for 1 hour under a current of nitrogen. After the mixture is allowed to stand for cooling, gel-like white crystals are precipitated. The crystals are dissolved in chloroform and subjected to a silica gel column (2.1×40 cm) chromatography, which is eluted with 0 to 10 % ethanol/dichloromethane solution (1100 ml). The fractions of the product are collected and the solvent is distilled off. The residue is crystallized wirh benzene to obtain white crystals (219 mg, 70 %). Also, a starting material (33 mg, 8.5 %) is recovered.

### (Example 14)

### Synthesis of 9-(5-O-trityl-2,3-dideoxy-2-fluoro-β-D-threo-pentofuranosyl) adenine; Part-4

9-(5-O-trityl-3-phenoxythiocarbonyloxy-2-deoxy-2-fluoro-β-D-arabinofuranosyl) adenine (386 mg, 0.63 mmol) is suspended in dry dioxane (8 ml), and to the mixture azobisisobutylonitrile (120 mg) and diphenyl methylsilane (1.2 ml) are added. The resulting mixture is stirred at 115 °C for 10 hours under nitrogen stream. After cooling, the solution is concentrated and the residue is dissolved in dichloromethane (10 ml) and subjected to a silica gel column (2.1×40 cm) chromatography, which is eluted with 0 to 10 % ethanol/dichloromethane solution (1100 ml). The fractions of the product are collected and the solvent is distilled off. The residue is crystallized with benzene to obtain white crystals (203 mg, 65 %). Also, a starting material (74 mg, 19 %) is recovered.

### (Example 15)

### Synthesis of 9-(2,3-dideoxy-2-fluoro-β-D-threopentofuranosyl) adenine (FddA)

9-(5-O-trityl-2,3-dideoxy-2-fluoro-β-D-threopentofuranosyl) adenine (300 mg, 0.605 mmol) is suspended in methanol (18 ml), and concentrated hydrochloric acid (1.2 ml) is added thereto. The mixture is stirred to dissolve it and further stirred for 1.5 hours at room temperature, and 4 ml of "Amberlite" (trademark) IR400B (OAc-type) are added thereto and stirred for 5 minutes. The reaction solution is analyzed by means of a thin layer chromatography and the spots based on FddA, the starting material and triphenylmethanol are obtained. A spot based on adenine is not found. The resin is removed by filtration, and thus obtained filtrate is concentrated to about 2 ml of solution, and then water (40 ml) is added thereto. The solution is washed three times with chloroform (10 ml), and the water layer is concentrated to obtain crystals in the form of prism yielding 116 mg in the first crystallization and 19 mg in the second crystallization. Total yield: 135 mg (FW: 253.24, 0.533 mmol, yield (ratio): 88 %).

### EFFECT OF INVENTION

In the process of the present invention, nucleoside derivatives wherein a fluorine atom, etc. has been substituted for the 2'-position thereof and the hydroxyl group at the 3'-position thereof has been deoxylated, such as 9-(2,3-dideoxy-2-fluoro-β-D-threo-pentofuranosyl) adenine (FddA), etc., which are used in the fields of the pharmaceutical products and the other products can be produced conveniently and industrially, and particularly a fluorin atom, etc. can be introduced to nucleoside derivatives at the 2'-position thereof in a high yield, and therefore the present invention provides an extremely useful and excellent process.

Further, processes for producing important and excellent intermediates usable when producing the above nucleoside derivatives and novel intermediates are also provided, as mentioned above:

## Claims

1. A process for producing a nucleoside derivative represented by the following general formula (4), which comprises subjecting a compound represented by the following general formula (2) to a Z group-substitution reaction step at the 6-position thereof and to a deprotection reaction step at the 3'-position thereof, to produce a compound represented by the following general formula (3), and subjecting the compound of formula (3) to a deoxylation reaction step at the 3'-position thereof: 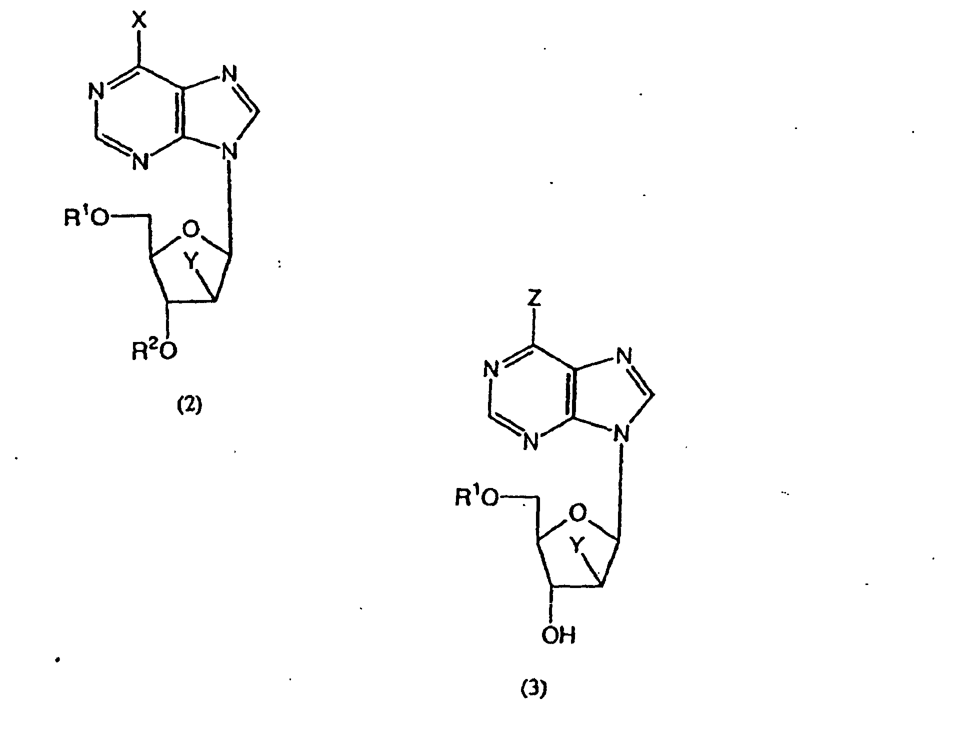 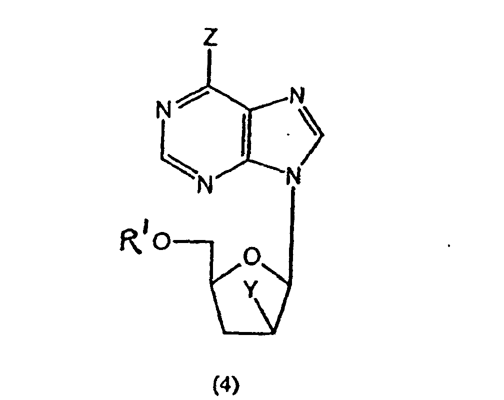 wherein X represents a halogen atom;
Y represents a substituent of anyone of a fluorine atom, an azide group and a cyano group;
Z represents any one of a hydrogen atom, an amino group, a hydroxyl group, an azide group, a
substituent of a formula OR⁴, a substituent of a formula SR⁴ and a substituent of a formula NHR⁴;
R¹ and R² represent protecting groups for a hydroxyl group which are different from each other such that conditions can be selected whereby one is selectively removable; and
R⁴ represents a lower alkyl group which may or may not have phenyl group(s) as the substituent group(s).

2. The process as defined in claim 1, which further comprises deprotecting the hydroxyl-protecting group at the 5'-position of compound (4) to produce a compound represented by the following general formula (8): 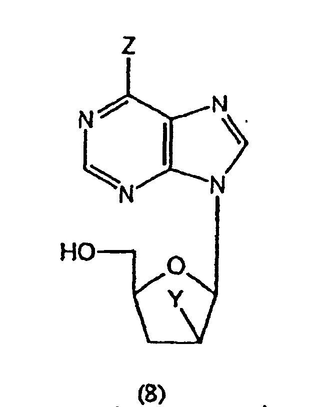 wherein Y and Z are as defined in claim 1.

3. The process as defined in claim 1 or 2, including a step of producing the compound represented by the general formula (2) by subjecting a compound represented by the following general formula (1) to a deoxylation/Y-substitution reaction step at the 2'-position thereof, or a step of subjecting a compound represented by the following general formula (1') to a removal of leaving group/Y-substitution reaction step at the 2'-position thereof: 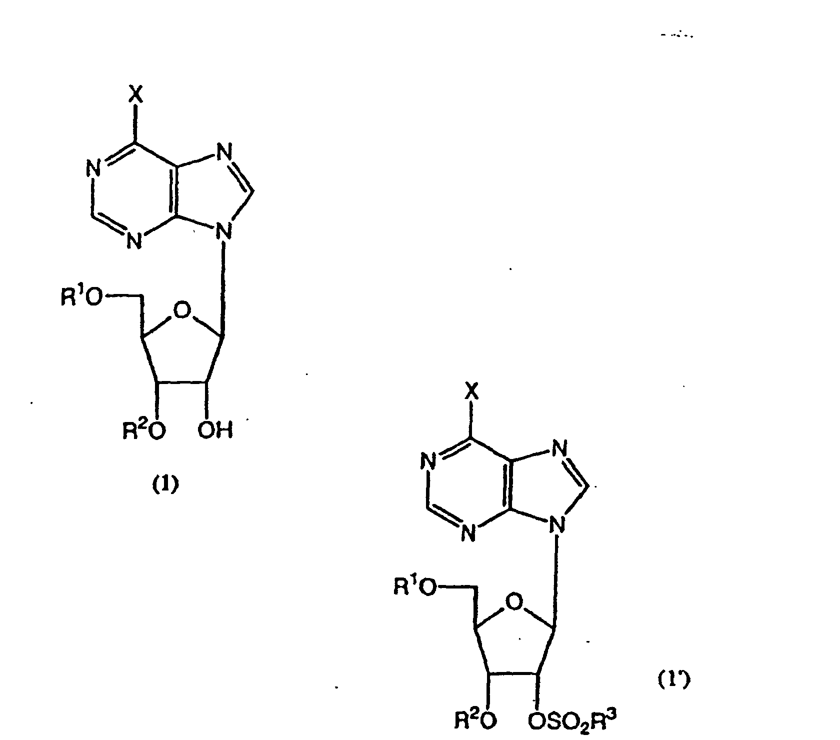 wherein X, Y, R¹ and R² are as defined in claim 1; and SO₂R³ represents a sulfonic acid-type leaving group.

4. The process as defined in claim 1, 2 or 3 wherein said Z group-substitution reaction step at the 6-position and said deprotection reaction step at the 3'-position contain an alkaline treatment step.

5. The process as defined in claim 4, wherein an alkaline material for said alkaline treatment is at least one selected from ammonia and alkaline water.

6. The process as defined in any preceding claim wherein Y represents a fluorine atom.

7. The process as defined in any preceding claim, wherein said Z group-substitution reaction is an amino group-substitution reaction, and in said formula (4), Z represents an amino group.

8. A process according to any preceding claim including preliminary and subsequent steps as defined in claims 3 and 2 to provide a process as shown in Scheme A: 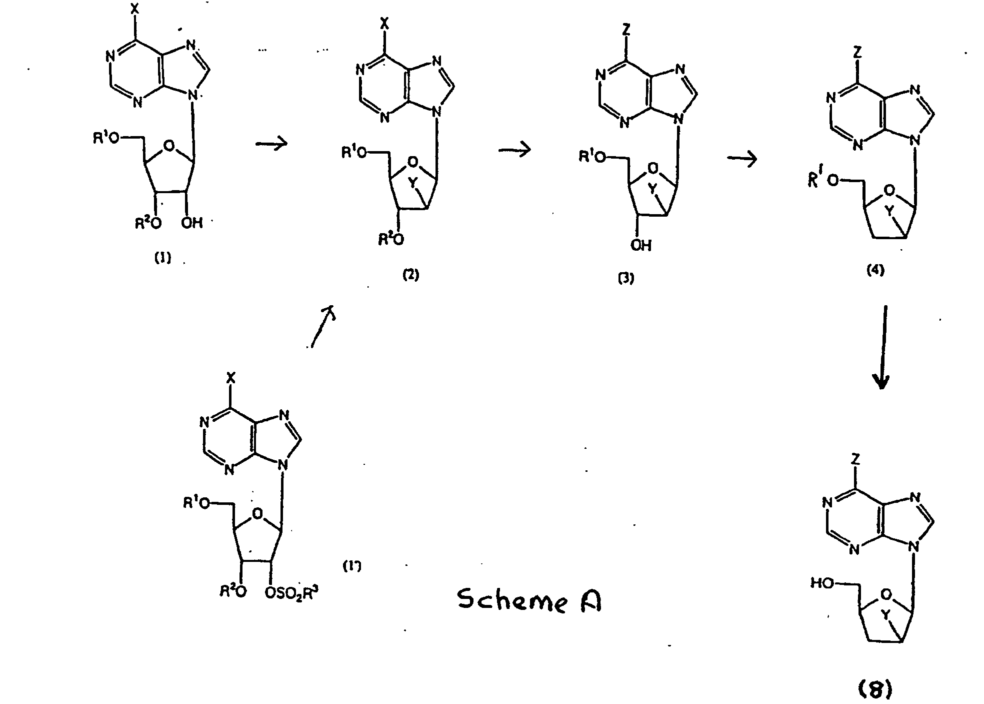 said process of Scheme A including a preliminary step comprising
(i) subjecting a compound represented by the general formula (1) to a deoxylation/Y-substitution reaction step at the 2'-position thereof; or
(ii) subjecting a compound represented by the following general formula (1') to a removal of leaving group/Y-substitution reaction step at the 2'-position thereof; said process of Scheme A including a subsequent step of deprotecting the hydroxyl group at the 5'-position to convert compound (4) into compound (8).

9. A process according to claim 8 wherein the preliminary step (ii) is preceded by
a step for producing the compound represented by the general formula (1'), which comprises: subjecting a compound represented by the general formula (1) to a reaction step for inserting a sulfonic acid-type leaving group thereinto.

10. A process for producing a nucleoside derivative comprising represented by the following general formula (3) the following steps:
(A) : a step for producing an intermediate represented by the following general formula (2), which comprises:
(i) subjecting a compound represented by the following general formula (1) to a deoxylation/Y-substitution reaction step at the 2'-position thereof; or
(ii) subjecting a compound represented by the following general formula (1) to a reaction step for inserting a sulfonic acid-type leaving group thereinto to produce a compound represented by the following general formula (1'); and subjecting the compound represented by the following general formula (1') to a removal of leaving group/Y-substitution reaction step at the 2'-position thereof;
(B) : a step which comprises: subjecting the compound represented by the following general formula (2) to a Z group-substitution reaction step at the 6-position thereof and to a deprotection step at the 3'-position thereof: 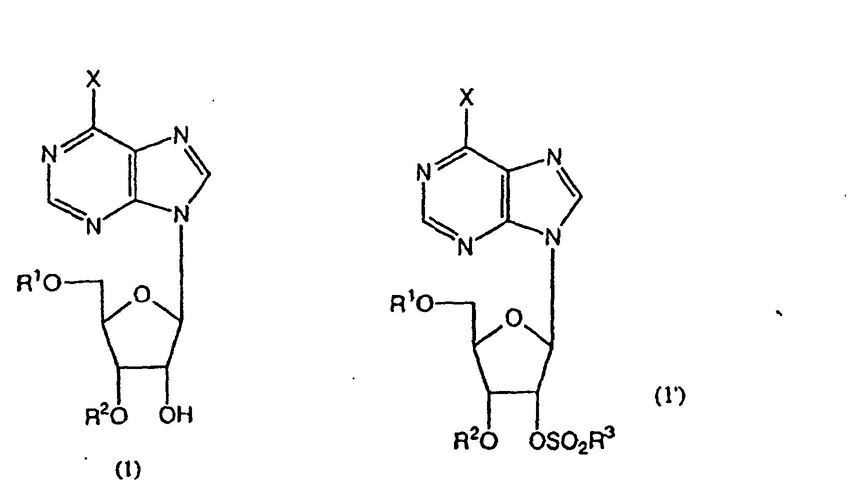 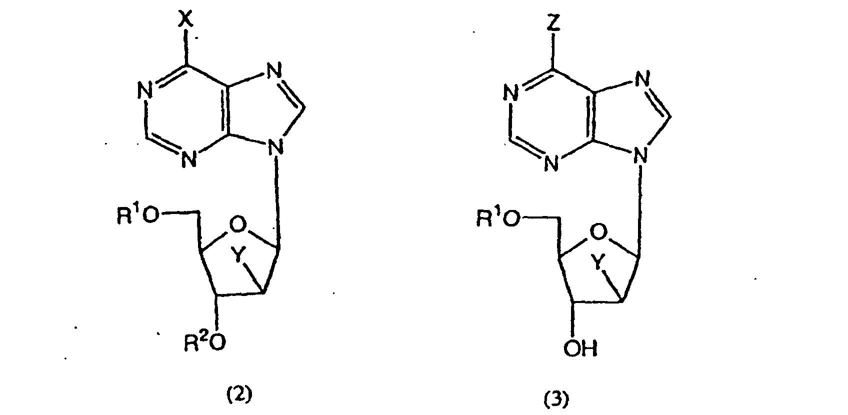
wherein X represents a halogen atom;
Y represents a substituent of any one of a fluorine atom, an azide group and a cyano group;
Z represents any one of a hydrogen atom, an amino group, a hydroxyl group, an azide group, a substituent of a formula OR⁴, a substituent of a formula SR⁴ and a substituent of a formula NHR⁴;
SO₂R³ represents a sulfonic acid-type leaving group; R¹ and R² represent protecting groups for a hydroxyl group which are different from each other such that conditions can be selected whereby one is selectively removable; and
R⁴ represents a lower alkyl group which may or may not have phenyl group(s) as the substituent group(s).

## Patentansprüche

1. Verfahren zur Herstellung eines Nukleosid-Derivats der folgenden allgemeinen Formel (4), umfassend das Unterziehen einer Verbindung der folgenden allgemeinen Formel (2) einem Z-Gruppen-Substitutitionsreaktionsschritt an deren 6-Position und einem Entschützungsreaktionsschritt an deren 3'-Position, um eine Verbindung der folgenden allgemeinen Formel (3) herzustellen, und Unterziehen der Verbindung der Formel (3) einem Deoxylierungsreaktionsschritt an deren 3'-Position: 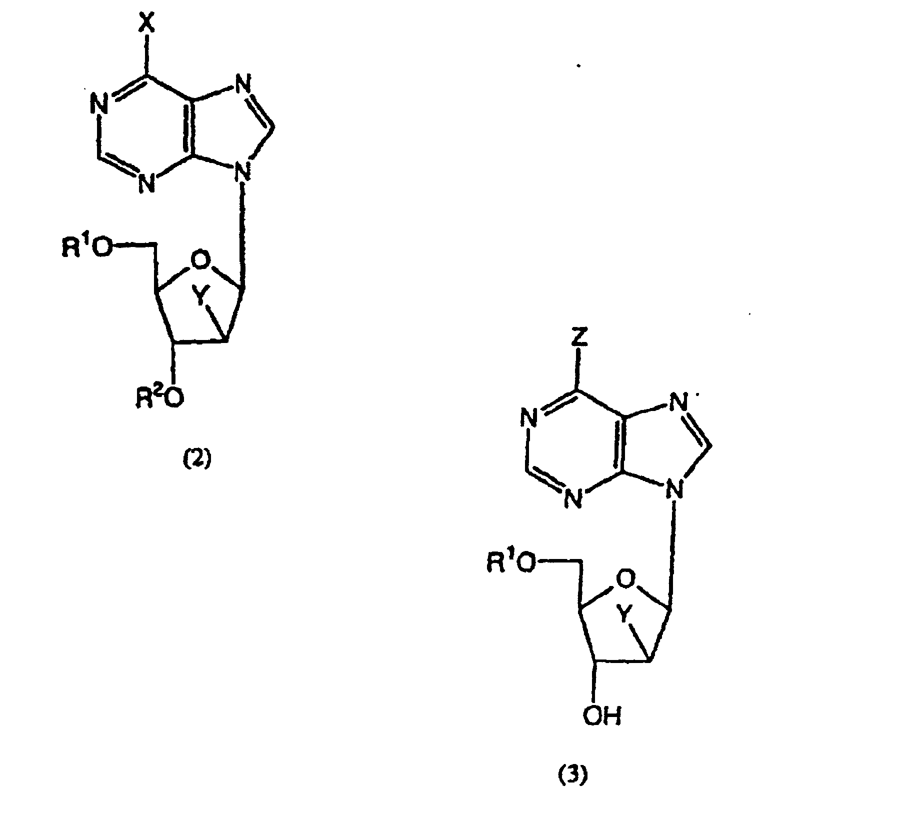 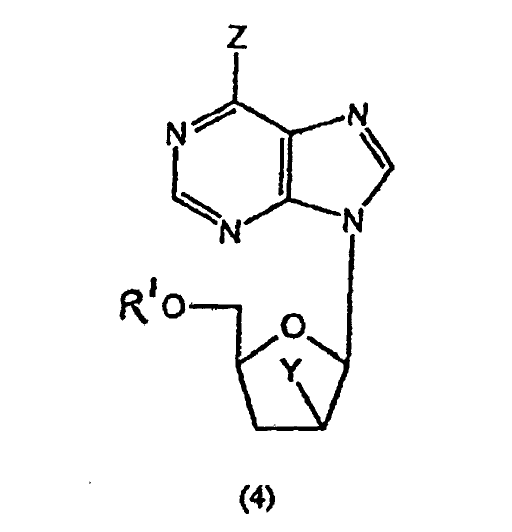 wobei X ein Halogenatom darstellt;
Y einen der Substituenten Fluoratom, Azidgruppe und Cyanogruppe darstellt;
Z ein Wasserstoffatom, eine Aminogruppe, eine Hydroxygruppe, eine Azidgruppe, einen Substituenten der Formel OR⁴, einen Substituenten der Formel SR⁴ oder einen Substituenten der Formel NHR⁴ darstellt,
R¹ und R² Schutzgruppen für eine Hydroxygruppe darstellen, die sich derart voneinander unterscheiden, dass Bedingungen gewählt werden können, unter denen eine selektiv entfernbar ist; und
R⁴ eine Niederalkylgruppe darstellt, die eine Phenylgruppe/Phenylgruppen als Substituentengruppe(n) aufweisen kann oder nicht.

2. Verfahren nach Anspruch 1, weiterhin umfassend das Entschützen der Hydroxyschutzgruppe an der 5'-Position von Verbindung (4), um eine Verbindung der folgenden allgemeinen Formel (8) herzustellen: 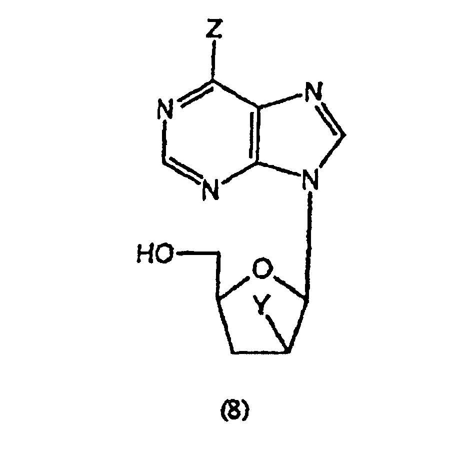 wobei Y und Z wie in Anspruch 1 definiert sind.

3. Verfahren wie in Anspruch 1 oder 2 definiert, einschließlich einem Schritt des Herstellens der Verbindung der allgemeinen Formel (2) durch Unterziehen einer Verbindung der folgenden allgemeinen Formel (1) einem Deoxylierungs-/Y-Substitutionsreaktionsschritt an deren 2'-Position, oder einem Schritt des Unterziehens einer Verbindung der folgenden allgemeinen Formel (1') einem Abgangsgruppenentfernungs-/Y-Substitutionsreaktionsschritt an deren 2'-Position: 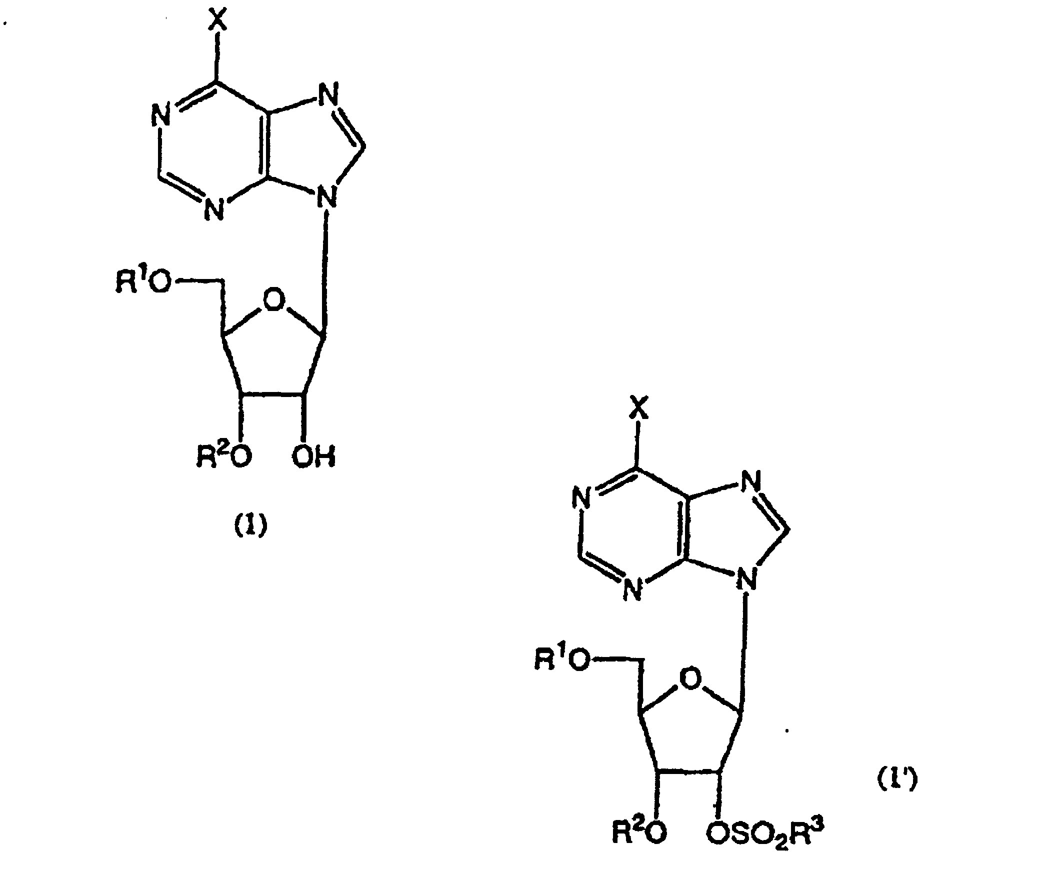 wobei X, Y, R¹ und R² wie in Anspruch 1 definiert sind, und SO₂R³ eine Abgangsgruppe vom Sulfonsäuretyp darstellt.

4. Verfahren nach Anspruch 1, 2 oder 3, wobei der Z-Gruppen-Substitutitionsreaktionsschritt an der 6-Position und der Entschützungsreaktionsschritt an der 3'-Position einen Alkalibehandlungsschritt beinhalten.

5. Verfahren nach Anspruch 4, wobei eine alkalische Substanz für die Alkalibehandlung mindestens eine solche ist, die aus Ammoniak und alkalischem Wasser ausgewählt ist.

6. Verfahren nach einem der vorhergehenden Ansprüche, wobei Y ein Fluoratom darstellt.

7. Verfahren nach einem der vorhergehenden Ansprüche, wobei die Z-Gruppen-Substitutionsreaktion eine Aminogruppensubstitutionsreaktion ist, und Z in der Formel (4) eine Aminogruppe darstellt.

8. Verfahren nach einem der vorhergehenden Ansprüche, einschließlich vorbereitender oder nachfolgender Schritte, wie in den Ansprüchen 3 und 2, so dass ein in Schema A gezeigtes Verfahren bereitgestellt wird, 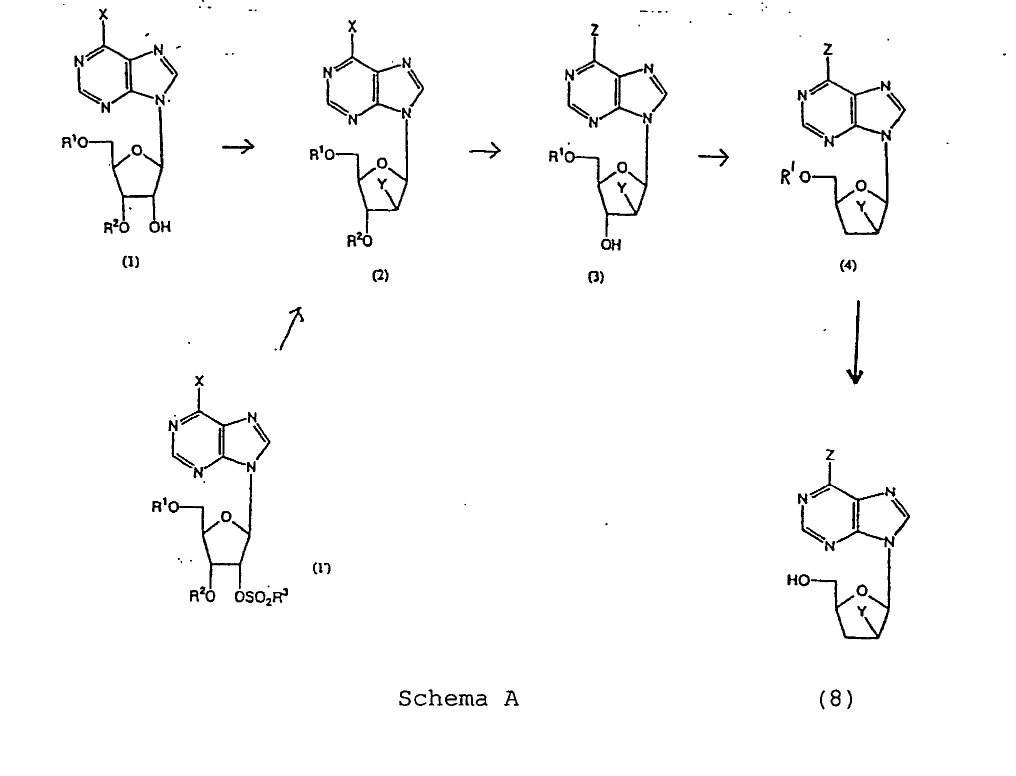 wobei das Verfahren von Schema A einen vorbereitenden Schritt beinhaltet, umfassend
(i) Unterziehen einer Verbindung der allgemeinen Formel (1) einem Deoxylierungs-/Y-Substitutionsreaktionsschritt an deren 2'-Position; oder
(ii) Unterziehen der folgenden allgemeinen Formel (1') einem Abgangsgruppenentfernungs-/Y-Substitutionsreaktionsschritt an deren 2'-Position, wobei das Verfahren von Schema A einen nachfolgenden Schritt des Entschützens der Hydroxygruppe an der 5'Position beinhaltet, um Verbindung (4) in Verbindung (8) umzuwandeln.

9. Verfahren nach Anspruch 8, wobei dem vorbereitenden Schritt (ii) ein Schritt des Herstellens der Verbindung der allgemeinen Formel (1') vorhergeht, umfassend: Unterziehen einer Verbindung der allgemeinen Formel (1) einem Reaktionsschritt zum Einbau einer Abgangsgruppe vom Sulfonsäuretyp in diese.

10. Verfahren zur Herstellung eines Nukleosidderivats der folgenden allgemeinen Formel (3) mit den folgenden Schritten:
(A): einen Schritt zur Herstellung eines Zwischenprodukts der folgenden allgemeinen Formel (2), umfassend:
(i) Unterziehen einer Verbindung der folgenden allgemeinen Formel (1) einem Deoxylierungs-/Y-Substitutionsreaktionsschritt an deren 2'-Position; oder
(ii) Unterziehen einer Verbindung der folgenden allgemeinen Formel (1) einem Reaktionsschritt zum Einbau einer Abgangsgruppe von Sulfonsäuretyp in diese, um eine Verbindung der folgenden allgemeinen Formel (1') herzustellen; und Unterziehen der Verbindung der folgenden allgemeinen Formel (1') einem Abgangsgruppenentfernungs-/Y-Substitutionsreaktionsschritt an deren 2'-Position;
(B): einen Schritt, umfassend:
Unterziehen der Verbindung der folgenden allgemeinen Formel (2) einem Z-Gruppen-Substitutitionsreaktionsschritt an deren 6-Position und einem Entschützungsschritt an deren 3'-Position: 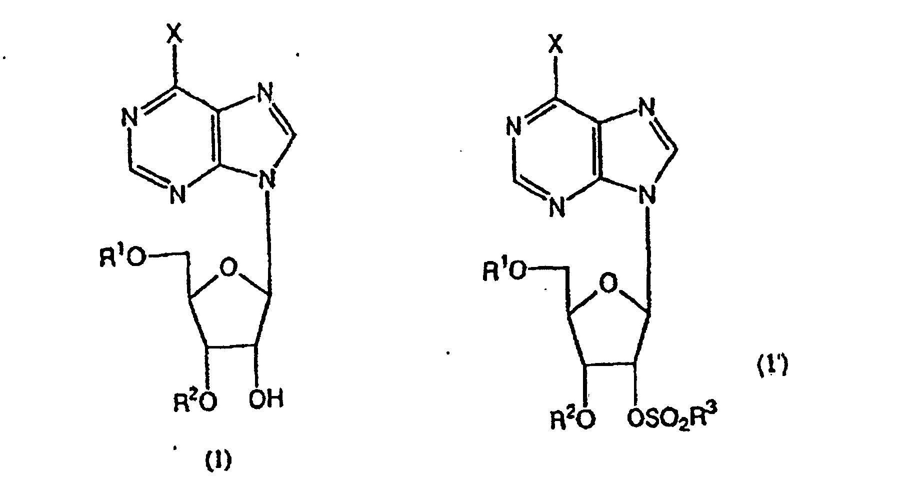 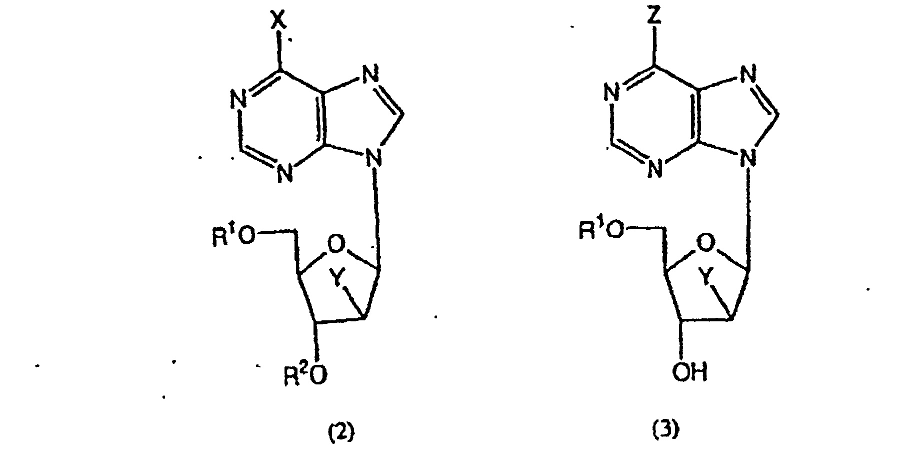 wobei X ein Halogenatom darstellt;
Y einen der Substituenten Fluoratom, Azidgruppe und Cyanogruppe darstellt;
Z ein Wasserstoffatom, eine Aminogruppe, eine Hydroxygruppe, eine Azidgruppe, einen Substituenten der Formel OR⁴, einen Substituenten der Formel SR⁴ oder einen Substituenten der Formel NHR⁴ darstellt;
SO₂R³ eine Abgangsgruppe vom Sulfonsäuretyp darstellt;
R¹ und R² Schutzgruppen für eine Hydroxygruppe darstellen, die sich derart voneinander unterscheiden, dass Bedingungen gewählt werden können, unter denen eine selektiv entfernbar ist; und
R⁴ eine Niederalkylgruppe darstellt, die eine Phenylgruppe/Phenylgruppen als die Substituentengruppe(n) aufweisen kann oder nicht.

## Revendications

1. Procédé pour produire un dérivé de nucléoside représenté par la formule générale (4) suivante, qui comprend la soumission d'un composé représenté par la formule générale (2) suivante à une étape de réaction de substitution de groupe Z à la position 6 de celui-ci et à une étape de réaction de déprotection à la position 3' de celui-ci, pour produire un composé représenté par la formule générale (3) suivante, et la soumission du composé de formule (3) à une étape de réaction de désoxylation à la position 3' de celui-ci : 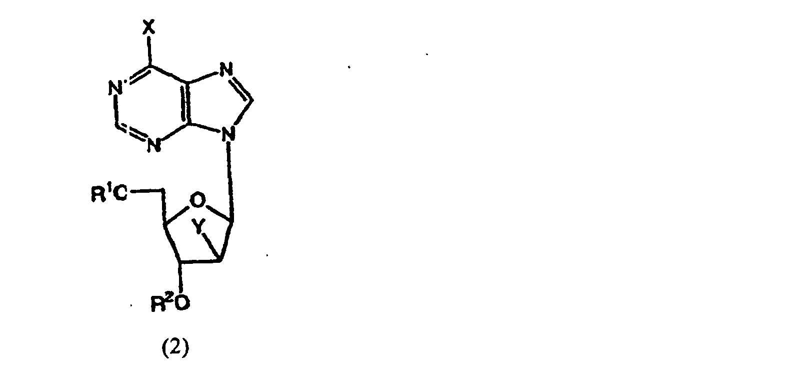 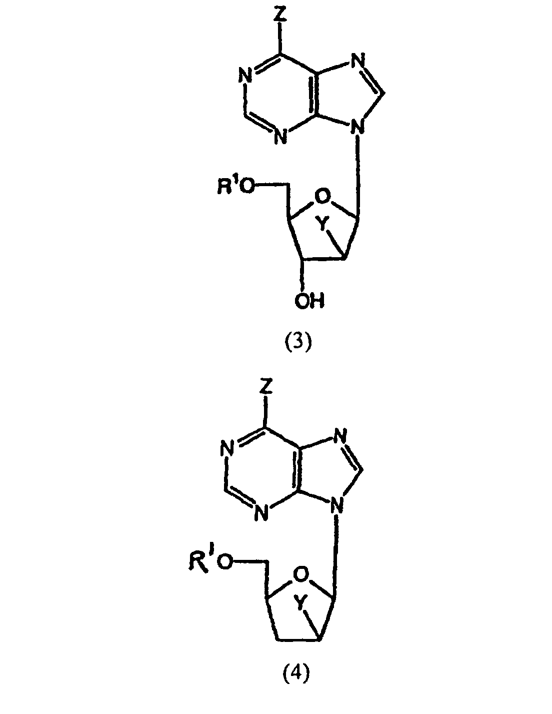 dans lesquels X représente un atome d'halogène ;
Y représente un substituant parmi l'un quelconque parmi un atome de fluor, un groupe azoture et un groupe cyano ;
Z représente l'un quelconque parmi un atome d'hydrogène, un groupe amino, un groupe hydroxyle, un groupe azoture, un substituant de formule OR⁴, un substituant de formule SR⁴ et un substituant de formule NNR⁴ ;
R² et R³ représentent des groupes protecteurs pour un groupe hydroxyle, qui sont différents l'un de l'autre de sorte que des conditions puissent être choisies de telle manière que l'un soit sélectivement éliminable ; et
R⁴ représente un groupe alkyle inférieur qui peut avoir ou non un ou des groupe(s) phényle en tant que groupe(s) substituant(s).

2. Procédé comme défini dans la revendication 1, qui comprend en outre la déprotection du groupe protecteur d'hydroxyle à la position 5' du composé (4) pour produire un composé représenté par la formule générale (8) suivante : 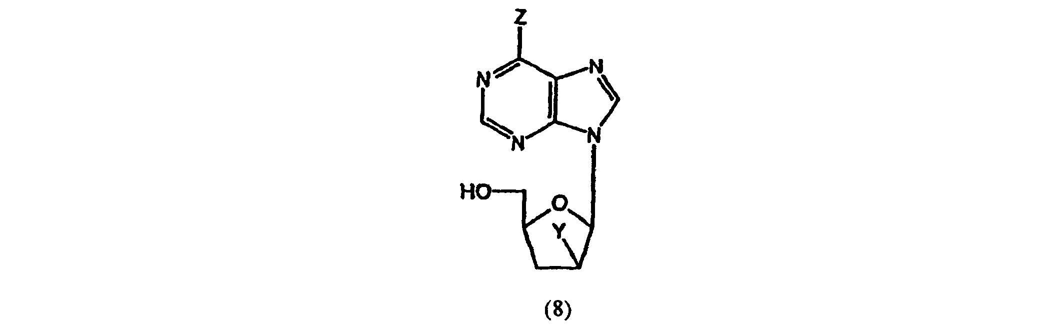 dans lequel Y et Z sont comme définis dans la revendication 1.

3. Procédé comme défini dans la revendication 1 ou 2, comprenant une étape de production du composé représenté par la formule générale (2) en soumettant un composé représenté par la formule générale (1) suivante à une étape de réaction de désoxylation/substitution de Y à la position 2' de celui-ci, ou une étape de soumission d'un composé représenté par la formule générale (1') suivante à une étape de réaction d'élimination de groupe labile/substitution de Y à la position 2' de celui-ci : 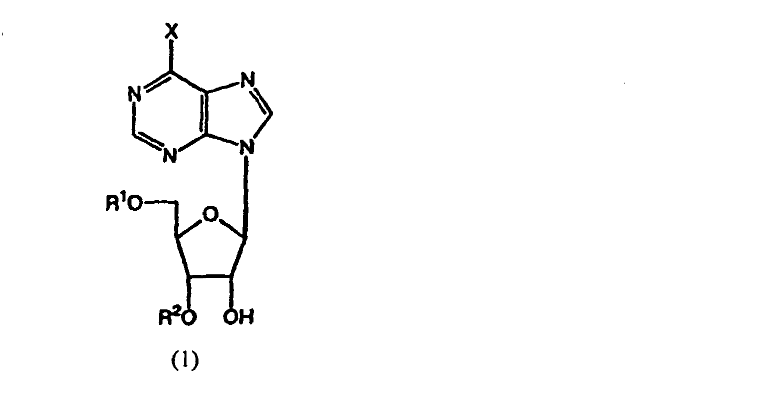 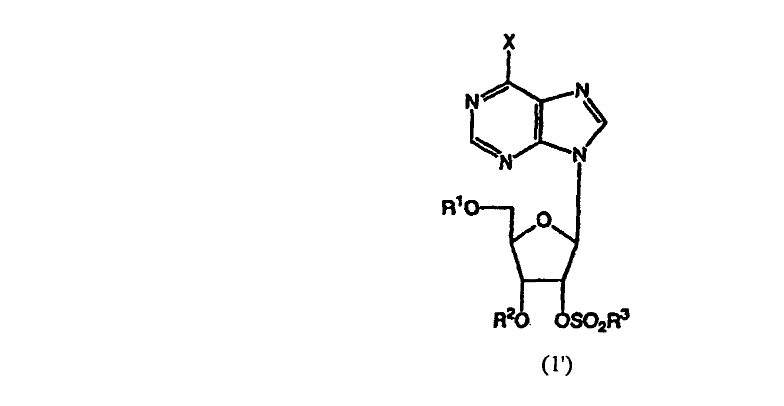 dans lesquels X, Y, R¹ et R² sont comme définis dans la revendication 1 ; et
SO₂R³ représente un groupe labile de type acide sulfonique.

4. Procédé comme défini dans la revendication 1, 2 ou 3, dans lequel ladite étape de réaction de substitution de groupe Z à la position 6 et ladite étape de réaction de déprotection à la position 3' comprennent une étape de traitement alcalin.

5. Procédé comme défini dans la revendication 4, dans lequel un matériau alcalin pour ledit traitement alcalin est au moins l'un choisi parmi l'ammoniaque et l'eau alcaline.

6. Procédé comme défini dans l'une quelconque des revendications précédentes dans lequel Y représente un atome de fluor.

7. Procédé comme défini dans l'une quelconque des revendications précédentes, dans lequel ladite réaction de substitution de groupe Z est une réaction de substitution de groupe amino, et dans ladite formule (4), Z représente un groupe amino.

8. Procédé selon l'une quelconque des revendications précédentes comprenant des étapes préliminaires et consécutives comme définies dans les revendications 3 et 2, pour produire un procédé comme décrit dans le schéma A : 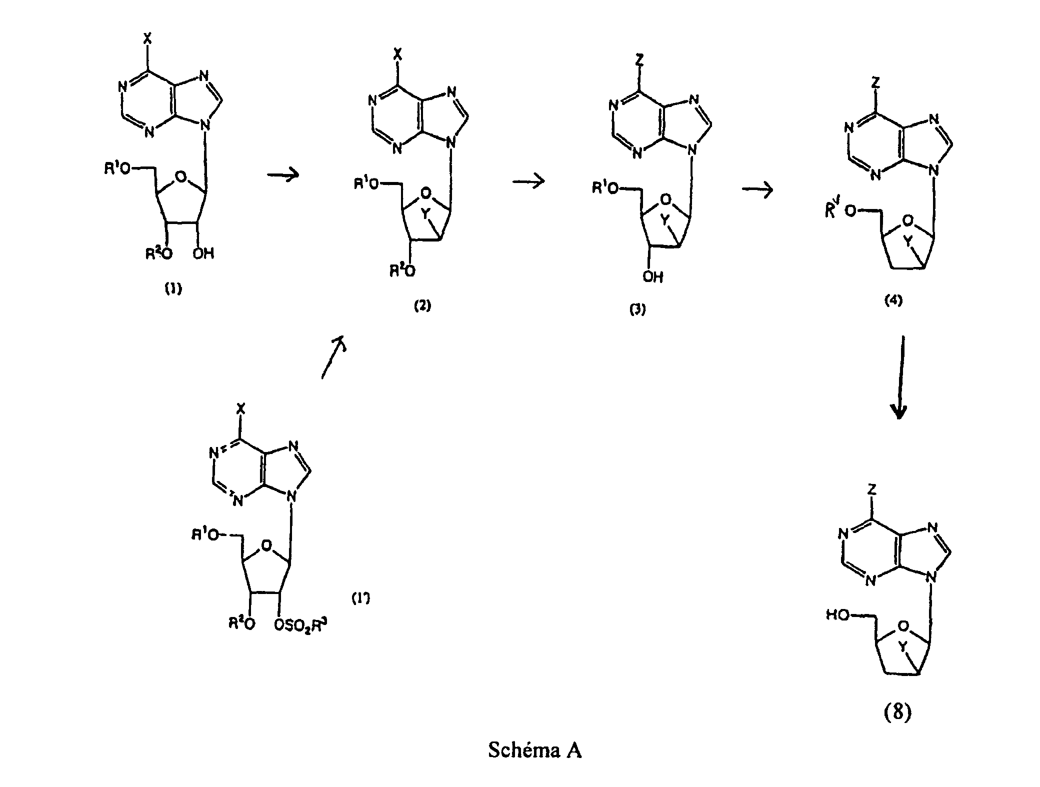 ledit procédé de schéma A comprenant une étape préliminaire comprenant
(i) la soumission d'un composé représenté par la formule générale (1) à une étape de réaction de désoxylation/substitution de Y à la position 2' de celui-ci ; ou
(ii) la soumission d'un composé représenté par la formule générale (1') suivante à une étape de réaction d'élimination de groupe labile/substitution de Y à la position 2' de celui-ci ;
ledit procédé de schéma A comprenant une étape consécutive de déprotection du groupe hydroxyle à la position 5' pour convertir le composé (4) en composé (8).

9. Procédé selon la revendication 8 dans lequel l'étape préliminaire (ii) est précédée par une étape pour produire le composé représenté par la formule générale (1') qui comprend la soumission d'un composé représenté par la formule générale (1) à une étape de réaction pour insérer un groupe labile de type acide sulfonique dans celui-ci.

10. Procédé pour produire un dérivé de nucléoside représenté par la formule générale (3) suivante comprenant les étapes suivantes :
(A) : étape pour produire un intermédiaire représenté par la formule générale (2) suivante, qui comprend :
(i) la soumission d'un composé représenté par la formule générale (1) suivante à une étape de réaction de désoxylation/substitution de Y à la position 2' de celui-ci ; ou
(ii) la soumission d'un composé représenté par la formule générale (1) suivante à une étape de réaction pour insérer un groupe labile de type acide sulfonique dans celui-ci pour produire un composé représenté par la formule générale (1') suivante ; et
la soumission du composé représenté par la formule générale (1') suivante à une étape de réaction d'élimination d'un groupe labile/substitution de Y à la position 2' de celui-ci ;
(B) : étape qui comprend : la soumission du composé représenté par la formule générale (2) suivante à une étape de réaction de substitution de groupe Z à la position 6 de celui-ci et à une étape de déprotection à la position 3' de celui-ci : 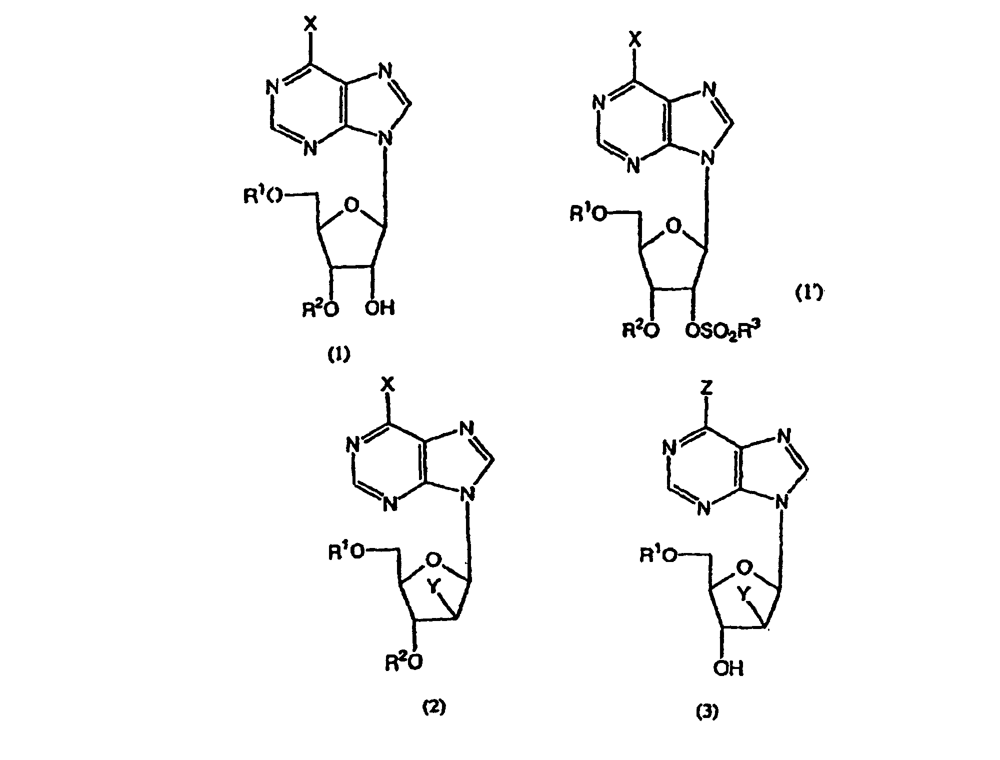
dans lesquels X représente un atome d'halogène ;
Y représente un substituant parmi l'un quelconque parmi un atome de fluor, un groupe azoture et un groupe cyano ;
Z représente l'un quelconque parmi un atome d'hydrogène, un groupe amino, un groupe hydroxyle, un groupe azoture, un substituant de formule OR⁴, un substituant de formule SR⁴ et un substituant de formule NHR⁴ ;
SO₂R³ représente un groupe labile de type acide sulfonique ;
R¹ et R² représentent des groupes protecteurs pour un groupe hydroxyle, qui sont différents l'un de l'autre de sorte que des conditions puissent être choisies de telle manière que l'un soit sélectivement éliminable ; et
R⁴ représente un groupe alkyle inférieur qui peut avoir ou non un ou des groupe(s) phényle en tant que groupe(s) substituant(s).
